(19) Europäisches Patentamt
European Patent Office
Office européen des brevets

(11) **EP 4 756 041 A1**

(12) **EUROPEAN PATENT APPLICATION**

(43) Date of publication:
**10.06.2026 Bulletin 2026/24**

(21) Application number: 24218311.9

(22) Date of filing: **09.12.2024**

(51) International Patent Classification (IPC):
***C12Q 1/6886*** (2018.01)

(52) Cooperative Patent Classification (CPC):
**C12Q 1/6886;** C12Q 2600/118; C12Q 2600/158

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AE Eindhoven (NL)**

(72) Inventor: **HOFFMANN, Ralf Dieter
Eindhoven (NL)**

(74) Representative: **Philips Intellectual Property &
Standards
High Tech Campus 52
5656 AG Eindhoven (NL)**

Remarks:
The complete document including Reference
Table(s) and the Sequence Listing(s) can be
downloaded from the EPO website

(54) **PREDICTING AN OUTCOME FOR PROSTATE CANCER PATIENTS**

(57) The invention relates to a method for predicting an outcome for a prostate cancer. Particularly the method is based on the expression levels of markers genes determined in a prostate cancer sample from the subject. The invention further relates to a kit for determining the gene expression levels and uses thereof in determining an outcome for a subject with prostate cancer. The invention also relates to therapies for use in the treatment of prostate cancer.

Fig. 1

EP 4 756 041 A1

**Description**

FIELD OF THE INVENTION

The invention relates to a method for predicting an outcome for a prostate cancer. Particularly the method is based on the expression levels of markers genes determined in a prostate cancer sample from the subject. The invention further relates to a kit for determining the gene expression levels and uses thereof in determining an outcome for a subject with prostate cancer. The invention also relates to therapies for use in the treatment of prostate cancer.

BACKGROUND OF THE INVENTION

Cancer is a class of diseases in which a group of cells displays uncontrolled growth, invasion and sometimes metastasis. These three malignant properties of cancers differentiate them from benign tumors, which are self-limited and do not invade or metastasize. Prostate Cancer (PCa) is the most commonly-occurring non-skin malignancy in men. It displays as a heterogeneous disease with varying potential to develop progressively to deadly forms of the disease. Of the estimated 417,000 annual new cases in Europe, around 92,000 will die from their disease (see Ferlay J. et al., GLOBOCAN 2012 v1.0, Cancer Incidence and Mortality Worldwide: IARC CancerBase No. 11 [Internet], Lyon, France, International Agency for Research on Cancer, 2013).

Clinically, various schemes for pre-surgical risk classification have been developed based upon longitudinal biological patient outcomes (see Rodrigues G. et al., "Pre-treatment risk stratification of prostate cancer patients: A critical review", Canadian Urological Association Journal, Vol. 6, No. 2, pages 121-127, 2012). While active surveillance (AS) is recommended by the various national and international guidelines for men with very low and low risk prostate cancer (see Mohler J. et al., "NCCN clinical practice guidelines in oncology: Prostate cancer, Version 1.2016", Journal of the National Comprehensive Cancer Network, Vol. 14, No. 1, pages 19-30, 2016), there is a significant sub-group in this patient population with a risk of 10 to 25% cancer recurrence after primary treatment (see, for example, Hernandez D.J. et al., "Contemporary evaluation of the D'Amico risk classification of prostate cancer", Journal of Urology, Vol. 70, No. 5, pages 931-935, 2007). These patients suffer from the burden of follow-up treatments that are typically triggered by biochemical relapse. Likewise, in the intermediate risk group there is a sub-population with low risk of biochemical progression (see, for example, Jung J.W. et al., "Stratification of patients with intermediate-risk prostate cancer", BJU International, Vol. 115, No. 6, pages 907-912, 2015). Nevertheless, this group is heterogeneous, comprising patients with varied outcomes, including those with aggressive pathological characteristics (see Abern M.R. et al., "Delayed radical prostatectomy for intermediate-risk prostate cancer is associated with biochemical recurrence: Possible implications for active surveillance from the SEARCH database", The Prostate, Vol. 73, No. 4, pages 409-417, 2013).

WO2019122037A1 describes a method of pre-surgical risk stratification of a prostate cancer subject, comprising determining a gene expression profile for phosphodiesterase 4D variant 7 (PDE4D7) in a biological sample obtained from the subject, determining an expression based risk score for the subject based on the gene expression profile, and determining a pre-surgical prognostic risk score for the subject based on the expression based risk score and pre-surgical clinical variables of the subject.

In patients who undergo treatment, the most important clinical prognostic indicators of disease outcome are the stage, pretherapy PSA level, and Gleason score. The higher the grade and the stage, the poorer the prognosis, however a subset of patients in the poor prognosis group can be successfully treated. Therefore improved methods for predicting a patient outcome, particularly for those patients with poor indicators, are needed. In addition, suitable treatment options are needed for the predicted outcomes.

These drawbacks, among others, are overcome by the products and methods as outlined in the appended claims.

SUMMARY OF THE INVENTION

In a first aspect the invention relates to a computer implemented method for predicting an outcome for a subject suffering from prostate cancer, the method comprising: receiving the result of the determined expression levels of PDE4D and ABCC5 in a prostate cancer sample obtained from the patient, and predicting the outcome for the subject based on the PDE4D and ABCC5 expression levels, wherein the outcome is a good outcome or a poor outcome.

In a second aspect the invention relates to a computer program product comprising instructions which, when the program is executed by a computer, cause the computer to carry out a method for predicting the outcome for a subject suffering from prostate cancer, based on the PDE4D and ABCC5 expression levels determined in a prostate cancer sample obtained from the subject, the method comprising: predicting the outcome for the subject based on the PDE4D and ABCC5 expression levels.

In a third aspect the invention relates to the use of a kit to determine an outcome for a subject suffering from prostate cancer, the use comprising using the kit to determine the expression levels of PDE4D and ABCC5 in a prostate cancer sample obtained from the subject, and determining the outcome for the subject based on the determined expression levels, wherein the kit comprises means for determining the expression levels of PDE4D and ABCC5 in a biological sample.

In a fourth aspect the invention relates to a therapy for use in the treatment of prostate cancer in a subject, the use comprising receiving the result of the determined expression levels of PDE4D and ABCC5 in a prostate cancer sample obtained from the subject, predicting the outcome for the subject based on the PDE4D and ABCC5 expression levels, wherein the outcome is a good outcome or a poor outcome, and administering the therapy when a poor outcome is predicted, wherein the therapy is selected from: radiation therapy, preferably ERBT, supplemented with ADT and one or more of Abiraterone, Apalutamide or Enzalutamide; or ADT supplemented with taxane based chemotherapy, preferably docetaxel, and supplemented with one of Abiraterone, Apalutamide or Enzalutamide; or one or more treatments selected from: PARP inhibitors, preferably Olaparib, Rucaparib, or Niraparib), in case the subject has mutations and/or variants in DNA repair genes or ERG gene translocations or TP53 mutations or loss; kinase inhibitors, preferably Gefitinib, Capivasertib, or Sorafenib) in case the subject has mutations in protein kinases; mTOR inhibitors, preferably Everolimus in case the subject has PTEN loss or PIK3CA mutations; or Immune Checkpoint inhibitors, preferably Pembroluzimab or Nivolumab in case the subject has high tumor mutational burden (TMB); wherein said treatment is optionally further comprises ADT and/or radiotherapy, preferably EBRT; preferably wherein the ADT is selected from; Luteinizing hormone-releasing hormone (LHRH) agonist alone, LHRH agonists combined with a first or second generation antiandrogen, or LHRH antagonists, preferably wherein the LHRH agonist is selected from Goserelin, Leuprolide, or Triptorelin, and/or the first generation antiandrogen is selected from Nilutamide, Flutamide, or Bicalutamide, and/or the second generation antiandrogen is selected from Apalutamide, Darolutamide, Enzalutamide, and Proxalutamide, and/or the LHRH antagonist is selected from Degarelix or Relugolix.

BRIEF DESCRIPTION OF THE DRAWINGS

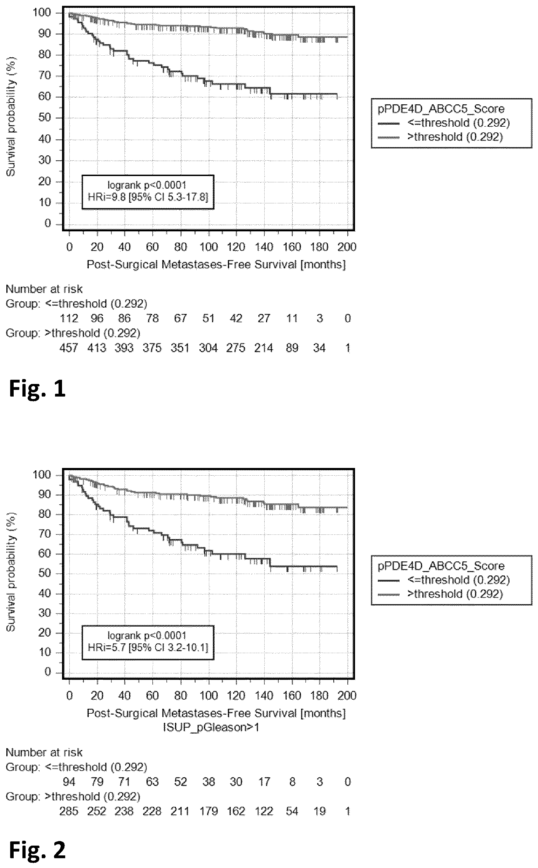

Fig. 1: depicts Post-Surgical Metastases-Free Survival, pPDE4D_ABCC5 Score (p -> percentile ranked scores of the PDE4D_ABCC5 Score) to predict Post-Surgical Metastases-Free Survival in patients with all ISUP Gleason scores on radical prostatectomy tissue. The threshold (0.292) was determined from the AUROC. HRi (inverse HR) and logrank p-value are given.

Fig. 2 depicts Post-Surgical Metastases-Free Survival, pPDE4D_ABCC5 Score (p -> percentile ranked scores of the PDE4D_ABCC5 Score) to predict Post-Surgical Metastases-Free Survival in patients with ISUP Gleason scores >1 on radical prostatectomy tissue. The threshold (0.292) was determined from the AUROC. HRi (inverse HR) and logrank p-value are given.

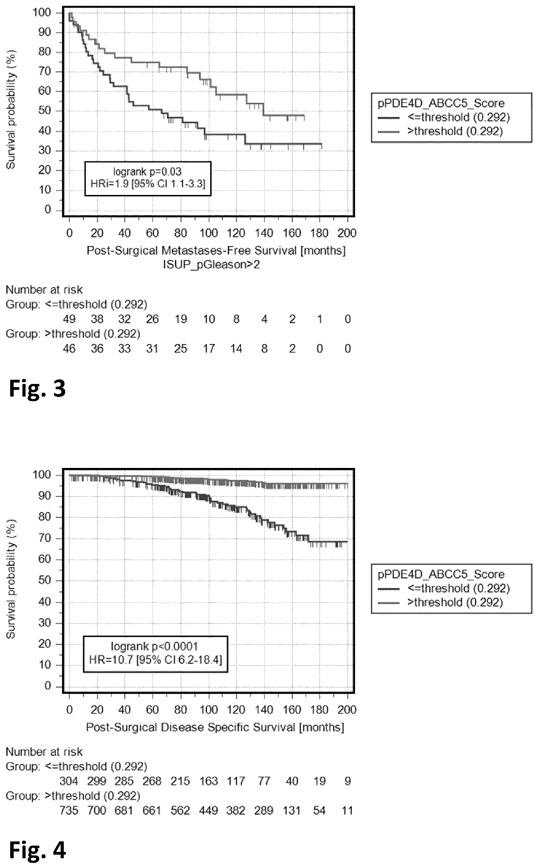

Fig. 3 depicts Post-Surgical Metastases-Free Survival, pPDE4D_ABCC5 Score (p -> percentile ranked scores of the PDE4D_ABCC5 Score) to predict Post-Surgical Metastases-Free Survival in patients with ISUP Gleason scores >2 on radical prostatectomy tissue. The threshold (0.292) was determined from the AUROC. HRi (inverse HR) and logrank p-value are given.

Fig. 4 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue. The threshold (0.292) was determined from the AUROC. HRi (inverse HR) and logrank p-value are given.

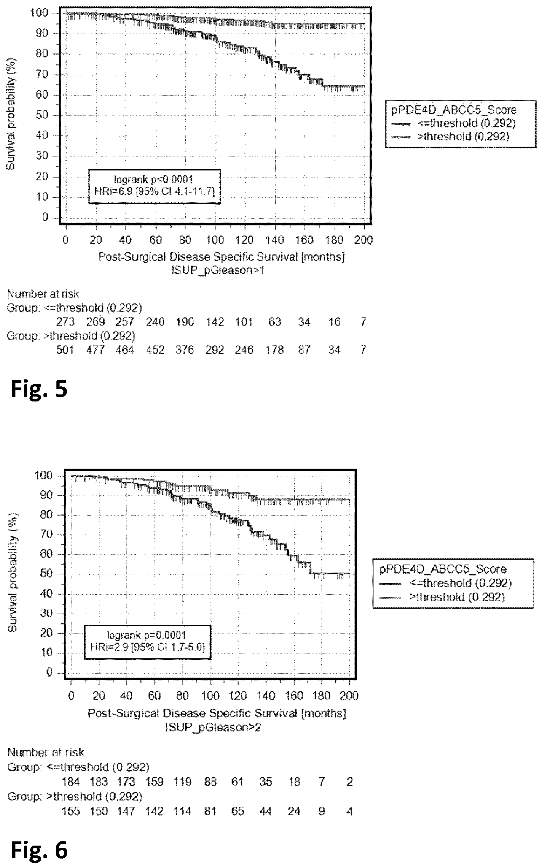

Fig. 5 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with ISUP Gleason scores >1 on radical prostatectomy tissue. The threshold (0.292) was determined from the AUROC. HRi (inverse HR) and logrank p-value are given.

Fig. 6 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with ISUP Gleason scores >2 on radical prostatectomy tissue. The threshold (0.292) was determined from the AUROC. HRi (inverse HR) and logrank p-value are given.

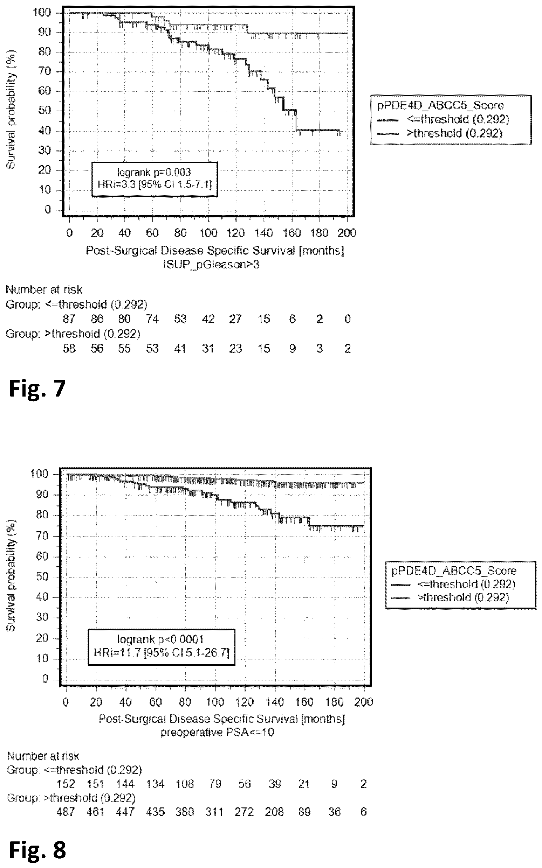

Fig. 7 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with ISUP Gleason scores >3 on radical prostatectomy tissue. The threshold (0.292) was determined from the AUROC. HRi (inverse HR) and logrank p-value are given.

Fig. 8 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pre=operative PSA = between 10 and 20 ng/ml. The threshold (0.292) was determined from the AUROC. HRi (inverse HR) and logrank p-value are given.

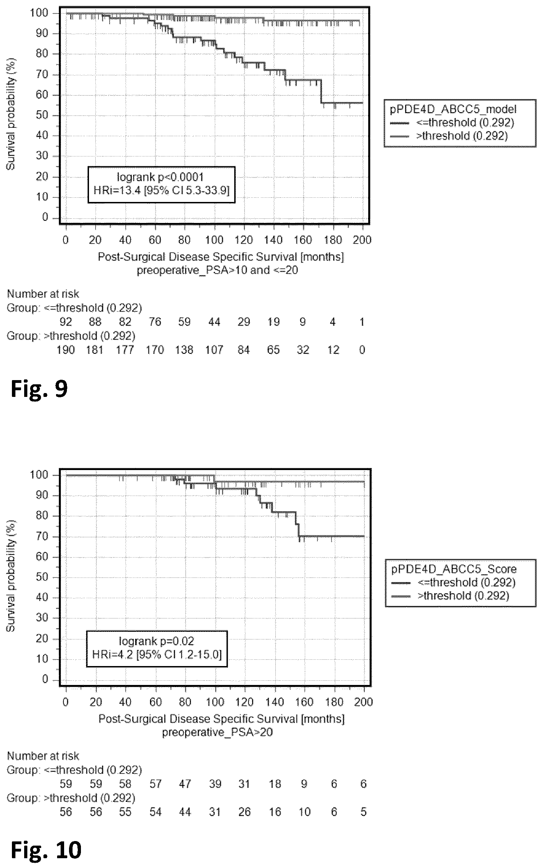

Fig. 9 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pre=operative PSA > 20 ng/ml. The threshold (0.292) was determined from the AUROC. HRi (inverse HR) and logrank p-value are given.

Fig. 10 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pre=operative PSA <=10 ng/ml. The threshold (0.292) was determined from the AUROC. HRi (inverse HR) and logrank p-value are given.

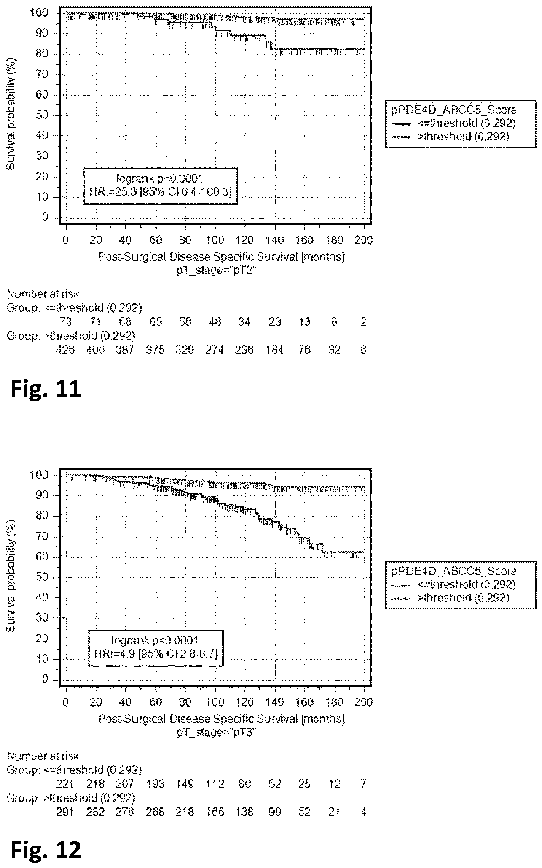

Fig. 11 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pathology pT stage pT2. The threshold (0.292) was determined from the AUROC. HRi (inverse HR) and logrank p-value are given.

Fig. 12 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pathology pT stage pT3. The threshold (0.292) was determined from the AUROC. HRi (inverse HR) and logrank p-value are given.

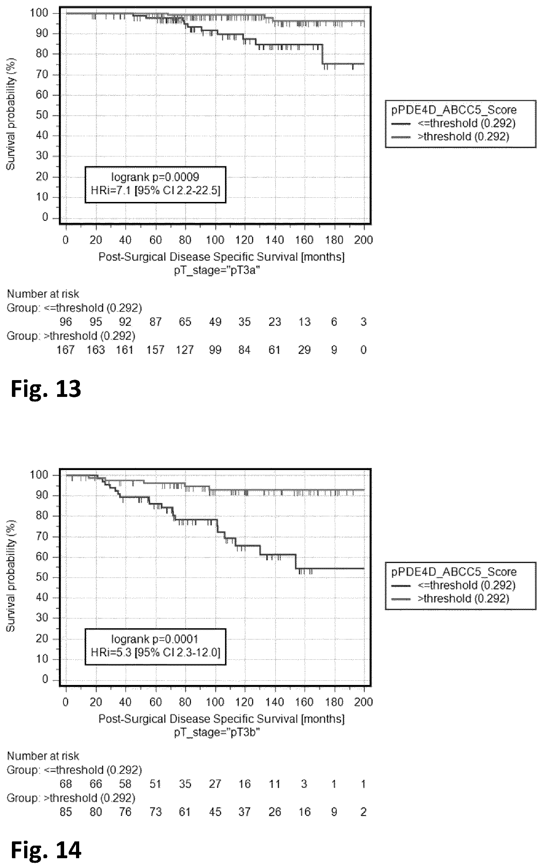

Fig. 13 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pathology pT stage pT3a. The threshold (0.292) was determined from the AUROC. HRi (inverse HR) and logrank p-value are given.

Fig. 14 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pathology pT stage pT3b. The threshold (0.292) was determined from the AUROC. HRi (inverse HR) and logrank p-value are given.

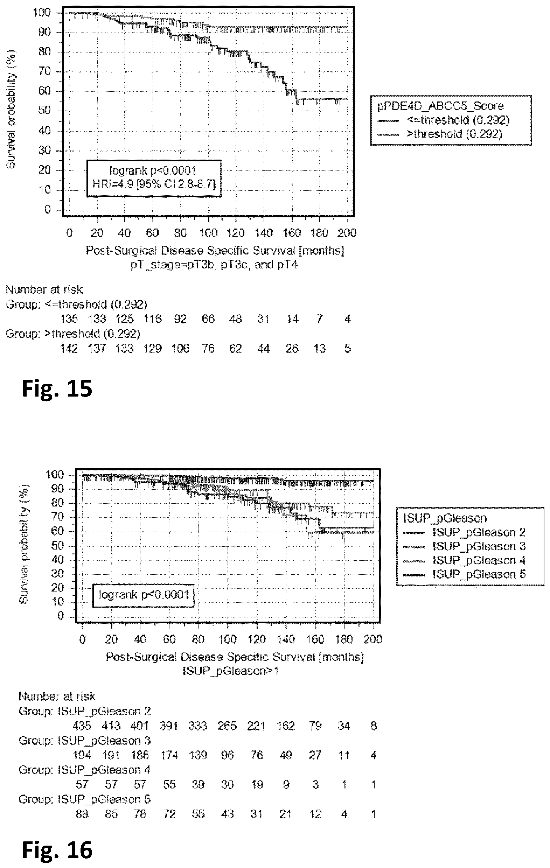

Fig. 15 depicts Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores on radical prostatectomy tissue and pathology pT stage >=pT3. The threshold (0.292) was determined from the AUROC. HRi (inverse HR) and logrank p-value are given.

Fig. 16 depicts Post-Surgical Prostate Cancer Specific Survival, ISUP pathology Gleason score to predict Post-Surgical Prostate Cancer specific survival in patients with ISUP Gleason scores >1 on radical prostatectomy tissue. The logrank p-value is given.

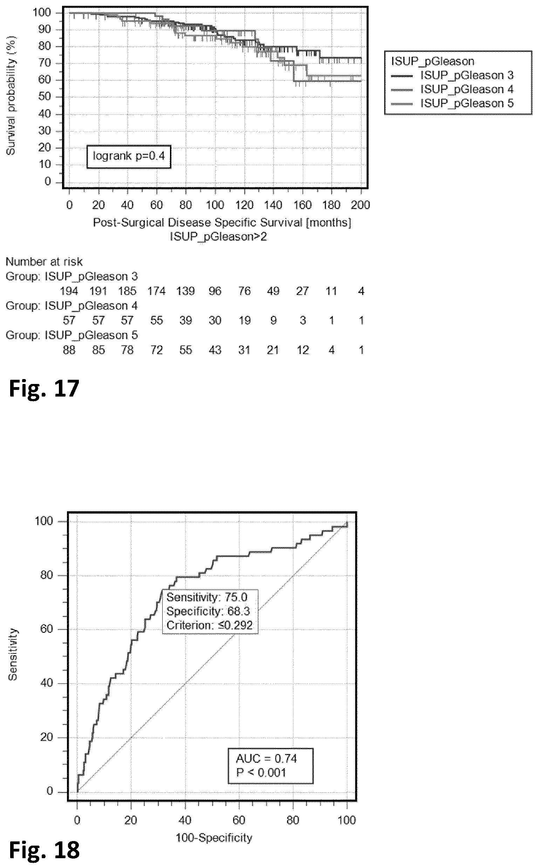

Fig. 17 depicts Post-Surgical Prostate Cancer Specific Survival, ISUP pathology Gleason score to predict Post-Surgical Prostate Cancer specific survival in patients with ISUP Gleason scores >2 on radical prostatectomy tissue. The logrank p-value is given.

Fig. 18 depicts AUROC of Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with all ISUP Gleason scores >1 on radical prostatectomy tissue. The threshold (0.292) was determined from the AUROC. The AUC and the p-value is given.

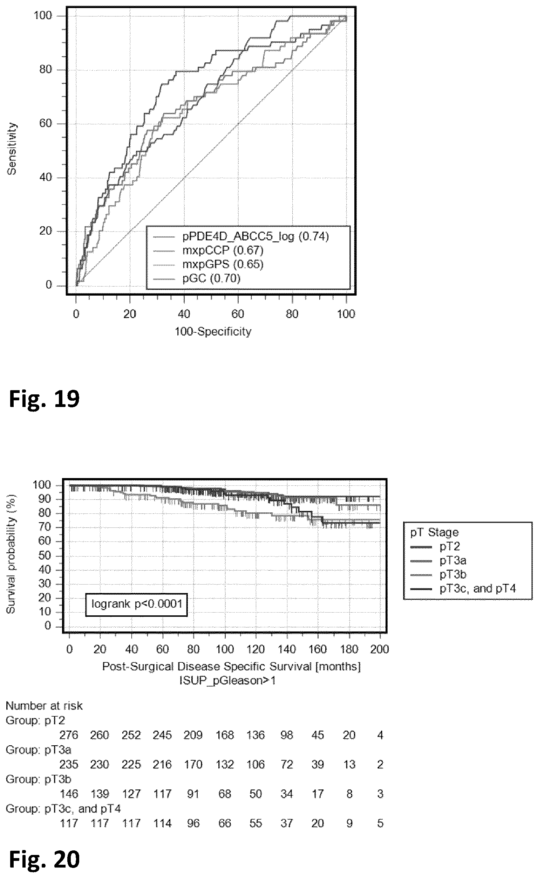

Fig. 19 depicts AUROC of Post-Surgical Prostate Cancer Specific Survival, pPDE4D_ABCC5 Score (p -> percentile ranked scores of the PDE4D_ABCC5 Score) to predict Post-Surgical Prostate Cancer specific survival in patients with ISUP Gleason scores >1 on radical prostatectomy tissue compared to Cell Cycle Prostate score (mxCCP), Genomic Prostate Score (mxGPS), Genomic Classifier (GC). The AUC and the p-value is given.

Fig. 20 depicts Post-Surgical Prostate Cancer Specific Survival, pT pathology stage to predict Post-Surgical Prostate Cancer specific survival in patients with ISUP Gleason scores >1 on radical prostatectomy tissue. The logrank p-value is given.

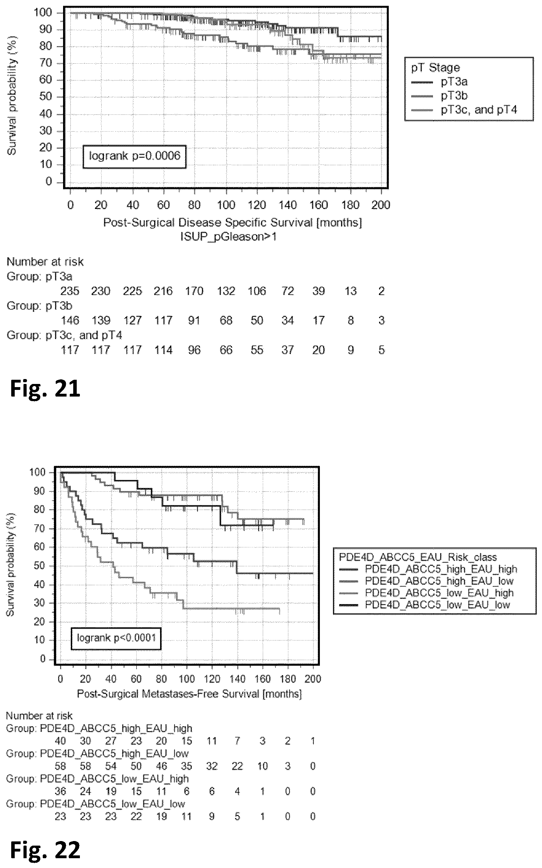

Fig. 21 depicts Post-Surgical Prostate Cancer Specific Survival, pT pathology stage >pT2 to predict Post-Surgical Prostate Cancer specific survival in patients with ISUP Gleason scores >1 on radical prostatectomy tissue. The logrank p-value is given.

Fig. 22 depicts Post-Surgical Metastases-Free Survival, the combined pPDE4D_ABCC5 Score (p -> percentile ranked scores of the PDE4D_ABCC5 Score) and EAU BCR Risk score to predict Post-Surgical Prostate Cancer specific survival in patients with all ISUP Gleason scores on radical prostatectomy tissue. The logrank p-value is given.

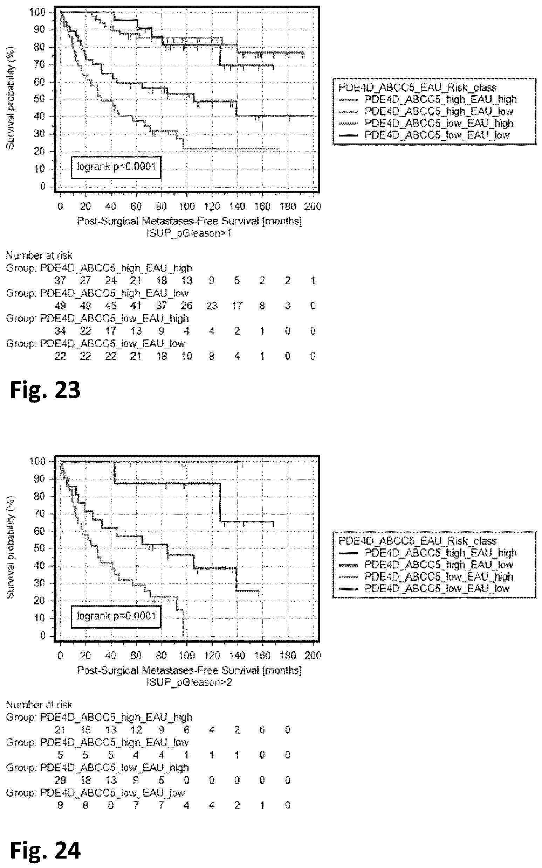

Fig. 23 depicts : Post-Surgical Metastases-Free Survival, the combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >1 on radical prostatectomy tissue. The logrank p-value is given.

Fig. 24 depicts Post-Surgical Metastases-Free Survival, the combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >2 on radical prostatectomy tissue. The logrank p-value is given.

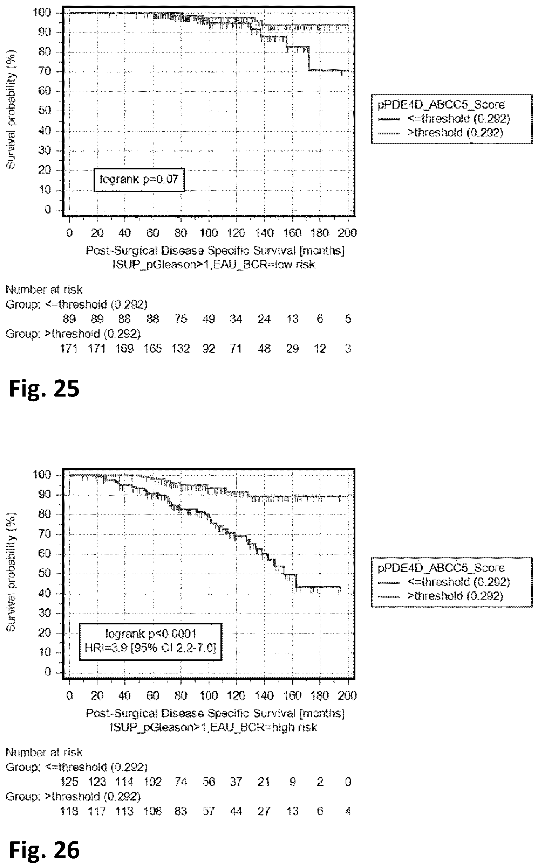

Fig. 25 depicts Post-Surgical Prostate Cancer Specific Survival, the pPDE4D_ABCC5 Score (p -> percentile ranked scores of the PDE4D_ABCC5 Score) tested on EAU BCR Risk score = 0 (i.e., EAU BCR Risk = low) to predict Post-Surgical Prostate Cancer specific survival in patients with ISUP Gleason scores >1 on radical prostatectomy tissue. The logrank p-value is given.

Fig. 26 depicts Post-Surgical Prostate Cancer Specific Survival, the pPDE4D_ABCC5 Score (p -> percentile ranked scores of the PDE4D_ABCC5 Score) tested on EAU BCR Risk score = 1 (i.e., EAU BCR Risk = high) to predict Post-Surgical Prostate Cancer specific survival in patients with ISUP Gleason scores >1 on radical prostatectomy tissue. The HRi and the logrank p-value is given.

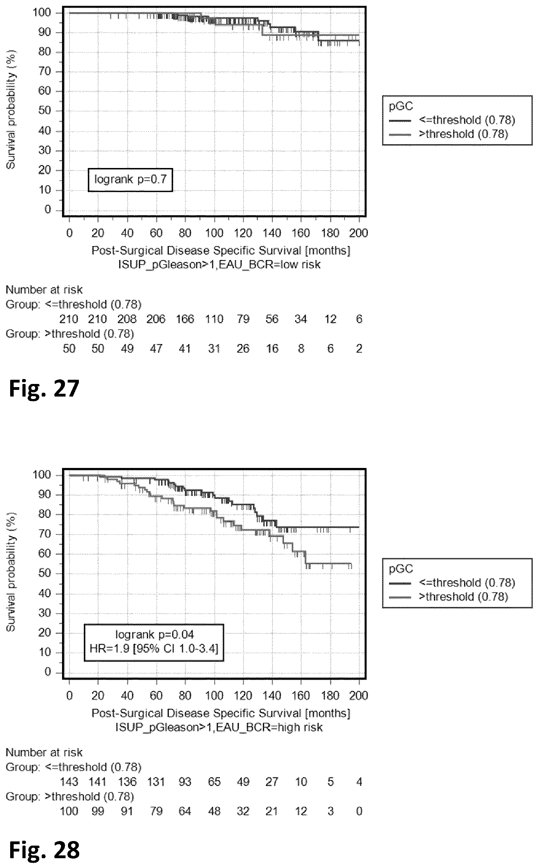

Fig. 27 depicts Post-Surgical Prostate Cancer Specific Survival, a prognostic gene classifier based on 18 genes of the 'Genomic Classifier' Score (p -> percentile ranked scores of the Genomic Classifier (GC) tested on EAU BCR Risk score = 0 (i.e., EAU BCR Risk = low) to predict Post-Surgical Prostate Cancer specific survival in patients with ISUP Gleason scores >1 on radical prostatectomy tissue. The logrank p-value is given.

Fig. 28 depicts Post-Surgical Prostate Cancer Specific Survival, a prognostic gene classifier based on 18 genes of the 'Genomic Classifier' Score (p -> percentile ranked scores of the Genomic Classifier (GC) tested on EAU BCR Risk score = 1 (i.e., EAU BCR Risk = high) to predict Post-Surgical Prostate Cancer specific survival in patients with ISUP Gleason scores >1 on radical prostatectomy tissue. The HR and logrank p-value is given.

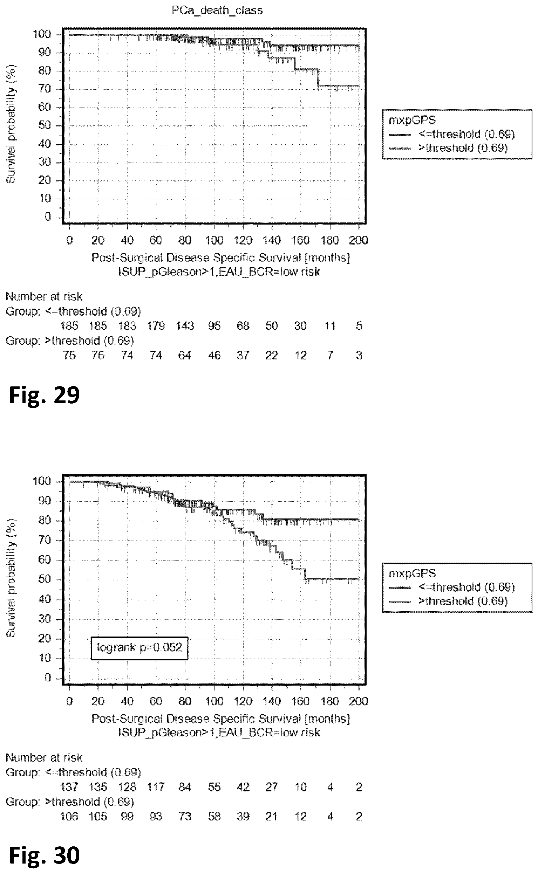

Fig. 29 depicts Post-Surgical Prostate Cancer Specific Survival, a prognostic gene classifier based on 17 genes of the 'Genomic Prostate Score' (mxp -> normalized, percentile ranked scores of the Genomic Prostate Score (GPS) score tested on EAU BCR Risk score = 0 (i.e., EAU BCR Risk = low) to predict Post-Surgical Prostate Cancer specific survival in patients with ISUP Gleason scores >1 on radical prostatectomy tissue. The logrank p-value is given.

Fig. 30 depicts Post-Surgical Prostate Cancer Specific Survival, a prognostic gene classifier based on 17 genes of the 'Genomic Prostate Score' (mxp -> normalized, percentile ranked scores of the Genomic Prostate Score (GPS) score tested on EAU BCR Risk score = 1 (i.e., EAU BCR Risk = high) to predict Post-Surgical Prostate Cancer specific survival in patients with ISUP Gleason scores >1 on radical prostatectomy tissue. The logrank p-value is given.

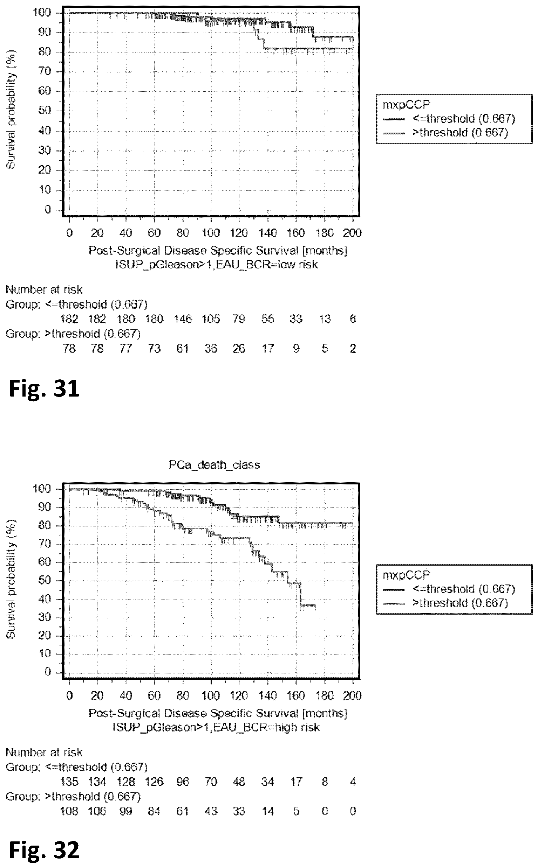

Fig. 31 depicts Post-Surgical Prostate Cancer Specific Survival, a prognostic gene classifier based on 46 genes of the 'Cell Cycle Progression' Score (mxp -> normalized, percentile ranked scores of the Cell Cycle Progression (CCP) score tested on EAU BCR Risk score = 0 (i.e., EAU BCR Risk = low) to predict Post-Surgical Prostate Cancer specific survival in patients with ISUP Gleason scores >1 on radical prostatectomy tissue. The logrank p-value is given.

Fig. 32 depicts Post-Surgical Prostate Cancer Specific Survival, a prognostic gene classifier based on 46 genes of the 'Cell Cycle Progression' Score (mxp -> normalized, percentile ranked scores of the Cell Cycle Progression (CCP) score tested on EAU BCR Risk score = 1 (i.e., EAU BCR Risk = high) to predict Post-Surgical Prostate Cancer specific survival in patients with ISUP Gleason scores >1 on radical prostatectomy tissue. The logrank p-value is given.

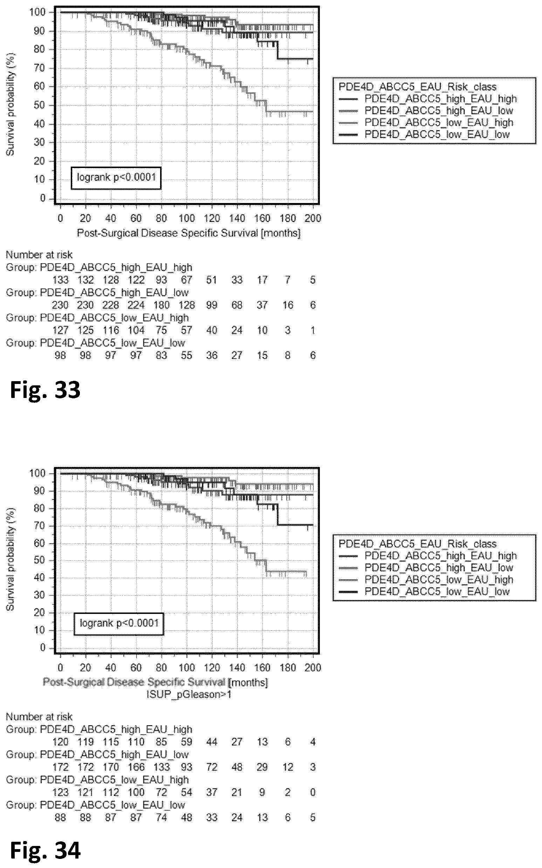

Fig. 33 depicts Post-Surgical Disease Specific Survival, the combined pPDE4D_ABCC5 Score (p -> percentile ranked scores of the PDE4D_ABCC5 Score) and EAU BCR Risk score to predict Post-Surgical Prostate Cancer specific survival in patients with all ISUP Gleason scores on radical prostatectomy tissue. The logrank p-value is given.

Fig. 34 depicts Post-Surgical Disease Specific Survival, the combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >1 on radical prostatectomy tissue. The logrank p-value is given.

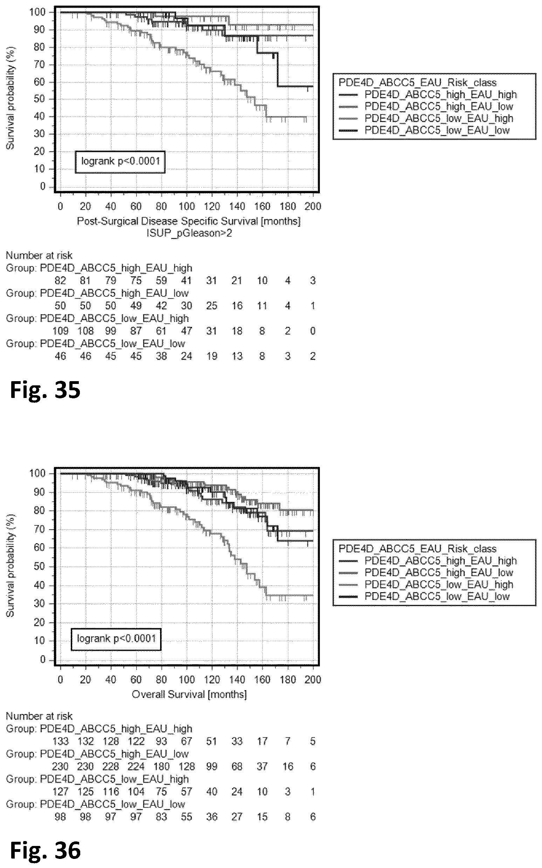

Fig. 35 depicts Post-Surgical Disease Specific Survival, the combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >2 on radical prostatectomy tissue. The logrank p-value is given.

Fig. 36 depicts Post-Surgical Overall Survival, The combined pPDE4D_ABCC5 Score (p -> percentile ranked scores of the PDE4D_ABCC5 Score) and EAU BCR Risk score to predict Post-Surgical Prostate Cancer specific survival in patients with all ISUP Gleason scores on radical prostatectomy tissue. The logrank p-value is given.

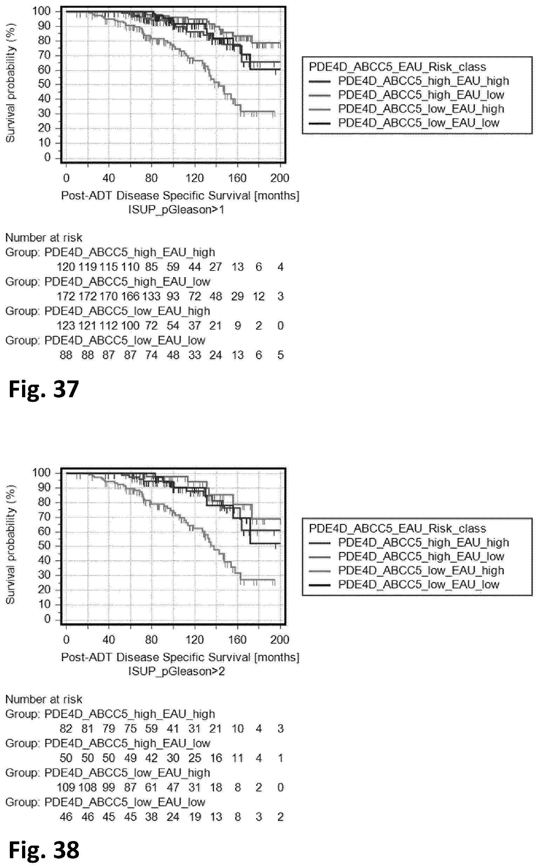

Fig. 37 depicts Post-ADT Disease specific Survival, the combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >1 on radical prostatectomy tissue. The logrank p-value is given.

Fig. 38 depicts Post-ADT disease specific Survival, The combined pPDE4D_ABCC5 Score (p -> percentile ranked scores of the PDE4D_ABCC5 Score) and EAU BCR Risk score to predict Post-Surgical Prostate Cancer specific survival in patients with ISUP Gleason scores >2 on radical prostatectomy tissue. The logrank p-value is given.

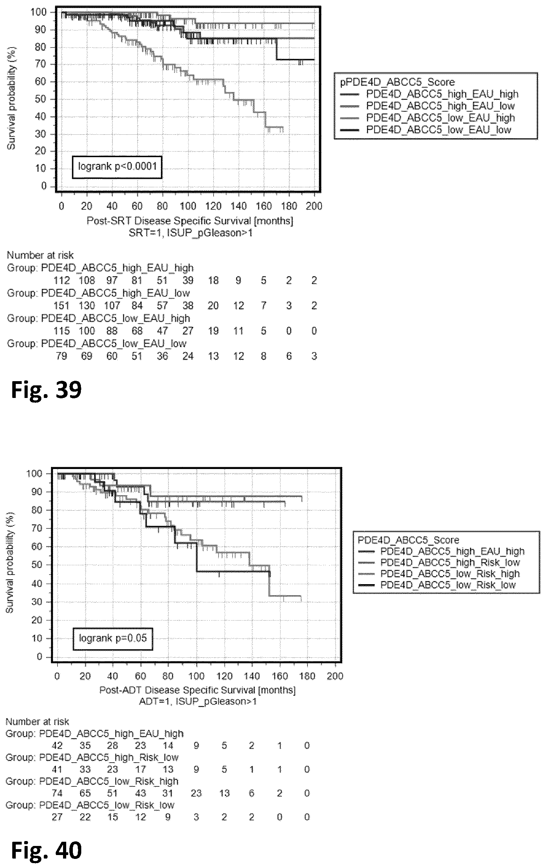

Fig. 39 depicts Post-SRT (Salvage Radiation Treatment) Disease Specific Survival, the combined pPDE4D_ABCC5 Score (p -> percentile ranked scores of the PDE4D_ABCC5 Score) and EAU BCR Risk score to predict Post-SRT Prostate Cancer specific survival in patients with ISUP Gleason scores >1 on radical prostatectomy tissue. The logrank p-value is given.

Fig. 40 depicts Post-SADT (Salvage Androgen Deprivation Treatment) Prostate Cancer Specific Survival, The combined pPDE4D_ABCC5 Score (p -> percentile ranked scores of the PDE4D_ABCC5 Score) and EAU BCR Risk score to predict Post-ADT Prostate Cancer specific survival in patients with ISUP Gleason scores >1 on radical prostatectomy tissue. The logrank p-value is given.

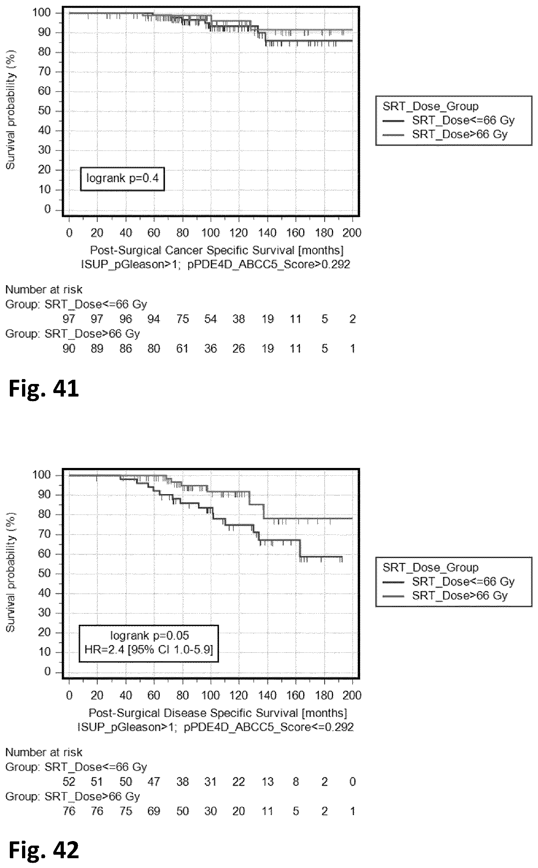

Fig. 41 depicts Post-Surgical Prostate Cancer Specific Survival, the SRT dose group (<=66 Gy vs >66 Gy total radiation dose) tested on the pPDE4D_ABCC5 Score (p -> percentile ranked scores of the PDE4D_ABCC5 Score) low risk to predict Post-Surgical Prostate Cancer specific survival in patients with ISUP Gleason scores >1 on radical prostatectomy tissue. The logrank p-value is given.

Fig. 42 depicts Post-Surgical Prostate Cancer Specific Survival, the SRT dose group (<=66 Gy vs >66 Gy total radiation dose) tested on the pPDE4D_ABCC5 Score (p -> percentile ranked scores of the PDE4D_ABCC5 Score) high risk to predict Post-Surgical Prostate Cancer specific survival in patients with ISUP Gleason scores >1 on radical prostatectomy tissue. The logrank p-value is given.

Definitions

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm20\%$, preferably $\pm15\%$, more preferably $\pm10\%$, and even more preferably $\pm5\%$.

"About" and "approximately": these terms, when referring to a measurable value such as an amount, a temporal duration, and the like, is meant to encompass variations of $\pm20\%$ or $\pm10\%$, more preferably $\pm5\%$, even more preferably $\pm1\%$, and still more preferably $\pm0.1\%$ from the specified value, as such variations are appropriate to perform the disclosed methods.

"Antagonist" and "inhibitor": These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

"Anti-cancer effect": This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

"Alleviating cancer": The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

"Compositions", "products" or "combinations": These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

"Combination therapy", or "in combination with": These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention. Unless otherwise defined, all technical and scientific terms used herein have the same meaning as commonly understood by a person of ordinary skill in the art.

The present invention broadly relates to the finding that PDE4D and ABCC5 expression levels when combined can be used to predict an outcome for a patient having prostate cancer. The method is particularly useful for intermediate to high risk patients, e.g. patients with an ISUP score of 3, 4, or 5, but also works for low risk patients, e.g. patients with an ISUP score of 1 or 2.

In order to demonstrate this the inventor has constructed a PDE4D_ABCC5 score based on the expression levels of PDE4D and ABCC5, and used a threshold for said score to differentiate between a good and a poor outcome. The examples show that doing so allows to distinguish between a good and poor outcome based on several different endpoints, such as post-surgical metastases free survival, post-surgical prostate cancer specific survival, overall survival, post-androgen deprivation therapy prostate cancer specific survival, post salvage radiation therapy prostate cancer specific survival, or post-surgical progression free survival. The effect is shown for a general population of prostate cancer patients, meaning all ISUP scores, but is also shown in sub-cohorts of high ISUP scores, for example 3 or more. The latter is particularly surprising and useful, as patients with a high ISUP score generally have a very poor outlook, so identifying patients with a good or poor outcome can be very useful to drive treatment decisions.

Although ISUP score alone can be used to determine an outcome in prostate cancer, it is difficult to distinguish outcomes in patients with ISUP scores of 3 or more, as for example shown in Reference Figs. 16 and 17. The same holds true for other cancer staging systems, as shown in Fig. 20 and 21, where pT pathology stage for more severe stages (pT3a, pT3b, pT3c, and pT4) does not distinguish adequately those patient at higher or lower risk.

The appended Examples and Figures show that based on PDE4D and ABCC5 expression levels a poor or good outcome can be predicted for a variation of clinical outcomes, such as disease specific survival, post-surgical disease specific survival, post-surgical prostate cancer specific survival, overall survival or post-ADT disease specific survival. The examples further show that such distinction between poor and good outcome is particularly prominent in higher ISUP grades (e.g. 3-5), in case of higher pre-operative PSA levels (e.g. 10 ng/ml or higher, or even 20 ng/ml or higher), higher pathology stages (e.g. pT3b, pT3c, or pT4).

When compared to other prognostic methods for prostate cancer outcome, the method presented here performs better (Figs. 18 and 19). Furthermore the data shows that the method can be combined with other methods (e.g. EAU BCR, GC, mxGPS, or mCCP) to further improve prediction.

Therefore in a first aspect the invention relates to a computer implemented method for predicting an outcome for a subject suffering from prostate cancer, the method comprising: receiving the result of the determined expression levels of PDE4D and ABCC5 in a prostate cancer sample obtained from the subject, and predicting the outcome for the subject based on the PDE4D and ABCC5 expression levels, wherein the outcome is a good outcome or a poor outcome.

The method may also comprise an active step of determining expression levels in the sample, therefore in an embodiment the invention relates to a implemented method for predicting an outcome for a subject suffering from prostate cancer, the method comprising: determining the expression levels of PDE4D and ABCC5 in a prostate cancer sample obtained from the subject, and predicting the outcome for the subject based on the PDE4D and ABCC5 expression levels, wherein the outcome is a good outcome or a poor outcome.

The expression levels are determined in a prostate cancer sample obtained from the subject. When used herein the term prostate cancer sample refers to a sample comprising at least one prostate cancer cell. The sample may for example be a tumor biopsy, part of a resected prostate, a tissue resection, or a blood sample comprising circulating tumor cells. In addition, the prostate cancer sample may include body tissue and/or a fluid, such as, but not limited to, blood (or blood derived such as serum, plasma, or PBMC's (peripheral blood mononuclear cells)), sweat, saliva, urine, bone marrow, and a needle biopsy or resection biopsy, preferably comprising at least one prostate cancer cell. Furthermore, the biological sample may contain a cell extract derived from or a cell population including a cancerous cell or a cell derived from tissue suspected to be cancerous, such as a myeloid cell. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumor cells, extracellular vesicles, a sample containing prostate secreted exosomes, or cell lines or cancer cell line.

In one particular realization, a prostate cancer sample such as a biopsy or resection sample may be obtained and/or used. Such samples may include prostate cancer cells or cell lysates.

It is also conceivable that the content of a prostate cancer sample is submitted to an enrichment step. For instance, a sample may be contacted with ligands specific for the cell membrane or organelles of certain cell types, e.g., tumor cells or myeloid cells, functionalized for example with magnetic particles. The material concentrated by the magnetic particles may subsequently be used for detection and analysis steps as described herein above or below.

Furthermore, prostate cancer cells, e.g., tumor cells, may be enriched via filtration processes of fluid or liquid samples, e.g., blood, urine, etc. Such filtration processes may also be combined with enrichment steps based on ligand specific interactions as described herein above.

When used herein the term prostate cancer cell refers to a tumor cell derived from the prostate or a metastasis thereof. It is preferred that a prostate cancer sample as provided herein is obtained from the subject before the start of the therapy. It is also preferred that said biological sample is a bladder sample or a bladder cancer sample. Thus, in a preferred embodiment the method according to the invention comprises that the prostate cancer sample is obtained from the subject before the start of the therapy, preferably wherein the prostate cancer sample is a prostate sample or a sample comprising prostate cancer cells from the subject. Alternatively, the method according to the invention comprises providing the prostate cancer sample obtained from the subject before the start of the therapy, preferably wherein the prostate cancer sample is a prostate sample or a sample comprising prostate cancer cells from the subject.

In an embodiment the prostate cancer sample is a resected prostate, a tumor biopsy, a prostate biopsy, a tissue resection, or any other sample obtained from the patient comprising at least one prostate cancer cell.

When used herein the term subject refers to a human or a non-human animal such as a mammal. When used herein the subject is a subject suffering from prostate cancer. The subject may be a patient, hence the terms subject and patient are used interchangeably herein. Preferably the subject has undergone or will undergo surgery. The surgery is preferably partial or complete removal of the prostate (radical prostatectomy), but the surgery may also be radiation therapy. In an embodiment the subject the subject has a pre-operative PSA level of 1 ng/ml or more, preferably 2, 3, 4,5, 6, 7, 8, 9, 10, 11, 12, 13, 14, 15, 16, 17, 18, 19or even 20 ng/ml or more. In an embodiment the subject has an ISUP score between 1-5, preferably 2-5, more preferably 3-5, most preferably 4 or 5. In an embodiment the subject has an ISUP score of 1, 2, 3, 4, or 5. In embodiment the subject has a pathology stage of pT2, pT3, pT3a, pT3b, pT3c, or pT4, preferably pT3, pT3a, pT3b, pT3c, or pT4, more preferably pT3b, pT3c, or pT4, most preferably pT3c, or pT4.

When used herein the pathology T stage is the stage of the tumor as determined on the resected prostate. Herein T1 means the cancer is too small to be seen on a scan, or felt during an examination of the prostate. It's divided into T1a, T1b and T1c, where T1a means that the cancer is in less than 5% of the removed tissue, T1b means that the cancer is in 5% or more of the removed tissue, and T1c cancers are found by biopsy, for example after a raised PSA level. pT2 means the cancer is completely inside the prostate gland. pT3 means the cancer has broken through the capsule of the prostate gland. It's divided into pT3a and pT3b, where T3a means the cancer has broken through the capsule of the prostate gland, and pT3b means the cancer has spread into the tubes that carry semen (seminal vesicles). pT4 means the cancer has spread into other body organs nearby, such as the back passage, bladder, or the pelvic wall.

Herein, the term "outcome" relates to a specific result or effect that can be measured. Examples of an outcome of a subject having prostate cancer include an outcome of the prostate cancer, a pathology outcome, an outcome of a therapy for treating prostate cancer, such as a ADT outcome or more specifically AADT, NADT or SADT outcome, as well as other outcomes, such as a biomarker related - e.g. biochemical recurrence, a genomic profile related outcome, an imaging related outcome (e.g., a change in morphology of the tumor), a biology related outcome (e.g., metastasis or recurrence of the tumor), a surrogate marker related outcome, a quality of life outcome, a cancer specific survival outcome, and an overall survival outcome.

The invention describes a prediction based on the expression levels of the genes PDE4D and ABCC5. These expression levels when combined are predictive for the outcome of a prostate cancer patient. In its simplest form a model can be constructed where each of PDE4D and ABCC5 individually are compared to a threshold to determine if the expression level is high or low. This may for example be done by setting a threshold expression level, above which the expression level is defined as high, and below which the expression level is defined as not high or low. Such threshold can easily be determined in a reference sample, or preferably a cohort of reference samples. For this the expression level is preferably quantified. Quantification may for example be done by normalizing to a household gene or by calculating reads per million, but other suitable method for quantifying gene expression levels are known in the art and are also be suitably used in the method described herein. Thus when used herein the term "high expression level" or "the expression level is high" can be interpreted as exceeding a threshold expression level for said gene. In this respect it is appreciated that a high expression level will be different for each gene and thus for each gene individually it needs to be determined what constitutes a high expression level, for example by establishing the appropriate threshold above which it is deemed high. The threshold may be determined using methods common in the art. For example from a pool of prostate cancer patients which includes patients having a poor outcome and patients having a good outcome (e.g. based in a clinical end point), expression levels may be collected for the respective gene and the threshold may be set as the mean or average expression level of this pool. Alternatively average expression levels may be determined for responders and non-responder and the threshold may be set as a value in between those average expression level values. The threshold is preferably chosen such as to optimize separation between the groups.

Thus when used herein a "value representing an expression level" refers to the quantification of said expression level reflected in a value. Such value may for example be expressed as transcripts per million or a normalized expression level. Expression levels may be normalized using one or more household genes. In one realization, the gene expression level for each of PDE4D, and ABCC5 is normalized with respect to one or more household genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these household genes.

Thus in an embodiment the expression levels thresholds for each of PDE4D, and ABCC5 are predetermined in a cohort of prostate cancer samples from patients.

When used herein the term "PDE4D" refers to the human cAMP-specific 3',5'-cyclic phosphodiesterase 4D gene (Ensembl: ENSG00000113448), for example, to the sequence as defined in NCBI Reference Sequence NM_001104631.2, specifically, to the nucleotide sequence as set forth in SEQ ID NO:32, which correspond to the sequences of the above indicated NCBI Reference Sequences of the PDE4D transcript, and also relates to the corresponding amino acid sequence for example as set forth in SEQ ID NO:33, which correspond to the protein sequences defined in NCBI Protein Accession Reference Sequence NP_001098101.1 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being at least 75%, 80%, 85%, 90%, 91%, 92%, 93%, 94%, 95%, 96%, 97%, 98% or 99% identical to the sequence as set forth in SEQ ID NO:33 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:32.

When used herein the term PDE4D may also refer to one or more of the specific described PDE4D isoforms, for example one or more of PDE4D1, PDE4D2, PDE4D3, PDE4D4, PDE4D5, PDE4D6, PDE4D7, PDE4D8 and PDE4D9. The when referring to PDE4D expression levels that expression levels may be the cumulative expression levels of two or more, for example two, three, four, five, six, seven, eight or all nine of the expression levels of individual isoforms selected from PDE4D1, PDE4D2, PDE4D3, PDE4D4, PDE4D5, PDE4D6, PDE4D7, PDE4D8 and PDE4D9.

The term "phosphodiesterase 4D1" or "PDE4D1" relates to the splice variant 1 of the human phosphodiesterase PDE4D, i.e. the human phosphodiesterase PDE4D1 gene, preferably to the sequence as defined in NCBI Reference Sequence: NM_001197222.1, more preferably to the nucleotide sequence as set forth in SEQ ID NO: 1, which corresponds to the sequence of the above indicated NCBI Reference Sequence of the PDE4D1 transcript, and also relates to the corresponding amino acid sequence as set forth in SEQ ID NO: 2, which corresponds to the protein sequence defined in NCBI Protein Accession Reference Sequence NP _001184151.1 encoding the PDE4D1 polypeptide. The term "phosphodiesterase 4D1" or "PDE4D1" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D2" or "PDE4D2" relates to the splice variant 2 of the human phosphodiesterase PDE4D, i.e. the human phosphodiesterase PDE4D2 gene, preferably to the sequence as defined in NCBI Reference Sequence: NM_001197221.1, more preferably to the nucleotide sequence as set forth in SEQ ID NO: 3, which corresponds to the sequence of the above indicated NCBI Reference Sequence of the PDE4D2 transcript, and also relates to the corresponding amino acid sequence as set forth in SEQ ID NO: 4, which corresponds to the protein sequence defined in NCBI Protein Accession Reference Sequence NP _001184150.1 encoding the PDE4D2 polypeptide. The term "phosphodiesterase 4D2" or "PDE4D2" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D3" or "PDE4D3" relates to the splice variant 3 of the human phosphodiesterase PDE4D, i.e. the human phosphodiesterase PDE4D3 gene, preferably to the sequence as defined in NCBI Reference Sequence: NM_006203.4, more preferably to the nucleotide sequence as set forth in SEQ ID NO: 5, which corresponds to the sequence of the above indicated NCBI Reference Sequence of the PDE4D3 transcript, and also relates to the corresponding amino acid sequence as set forth in SEQ ID NO: 6, which corresponds to the protein sequence defined in NCBI Protein Accession Reference Sequence NP_006194.2 encoding the PDE4D3 polypeptide.

The term "phosphodiesterase 4D4" or "PDE4D4" relates to the splice variant 4 of the human phosphodiesterase PDE4D, i.e. the human phosphodiesterase PDE4D4 gene, preferably to the sequence as defined in NCBI Reference Sequence: NM_001104631.1, more preferably to the nucleotide sequence as set forth in SEQ ID NO: 7, which corresponds to the sequence of the above indicated NCBI Reference Sequence of the PDE4D4 transcript, and also relates to the corresponding amino acid sequence as set forth in SEQ ID NO: 8, which corresponds to the protein sequence defined in NCBI Protein Accession Reference Sequence NP_001098101.1 encoding the PDE4D4 polypeptide.

The term "phosphodiesterase 4D5" or "PDE4D5" relates to the splice variant 5 of the human phosphodiesterase PDE4D, i.e. the human phosphodiesterase PDE4D5 gene, preferably to the sequence as defined in NCBI Reference Sequence: NM_001197218.1, more preferably to the nucleotide sequence as set forth in SEQ ID NO: 9, which corresponds to the sequence of the above indicated NCBI Reference Sequence of the PDE4D5 transcript, and also relates to the corresponding amino acid sequence as set forth in SEQ ID NO: 10, which corresponds to the protein sequence defined in NCBI Protein Accession Reference Sequence NP _001184147.1 encoding the PDE4D5 polypeptide. The term "phosphodiesterase 4D5" or "PDE4D5" also relates to the amplicon that can be generated by the primer pair PDE1D5_forward (SEQ ID NO: 23) and the PDE1D5_reverse (SEQ ID NO: 24) and can be detected by probe SEQ ID NO: 25.

The term "phosphodiesterase 4D6" or "PDE4D6" relates to the splice variant 6 of the human phosphodiesterase PDE4D, i.e. the human phosphodiesterase PDE4D6 gene, preferably to the sequence as defined in NCBI Reference Sequence: NM_001197223.1, more preferably to the nucleotide sequence as set forth in SEQ ID NO: 11, which corresponds to the sequence of the above indicated NCBI Reference Sequence of the PDE4D6 transcript, and also relates to the corresponding amino acid sequence as set forth in SEQ ID NO: 12, which corresponds to the protein sequence defined in NCBI Protein Accession Reference Sequence NP _001184152.1 encoding the PDE4D6 polypeptide.

The term "phosphodiesterase 4D7" or "PDE4D7" relates to the splice variant 7 of the human phosphodiesterase PDE4D, i.e. the human phosphodiesterase PDE4D7 gene, preferably to the sequence as defined in NCBI Reference Sequence: NM_001165899.1, more preferably to the nucleotide sequence as set forth in SEQ ID NO: 13, which corresponds to the sequence of the above indicated NCBI Reference Sequence of the PDE4D7 transcript, and also relates to the corresponding amino acid sequence as set forth in SEQ ID NO: 14, which corresponds to the protein sequence defined in NCBI Protein Accession Reference Sequence NP_001159371.1 encoding the PDE4D7 polypeptide. The term "phosphodiesterase 4D7" or "PDE4D7" also relates to the amplicon that can be generated by the primer pair PDE1D7_forward (SEQ ID NO: 26) and the PDE1D7_reverse (SEQ ID NO: 27) and can be detected by probe SEQ ID NO: 28.

The term "phosphodiesterase 4D8" or "PDE4D8" relates to the splice variant 8 of the human phosphodiesterase PDE4D, i.e. the human phosphodiesterase PDE4D8 gene, preferably to the sequence as defined in NCBI Reference Sequence: NM_001197219.1, more preferably to the nucleotide sequence as set forth in SEQ ID NO: 15, which corresponds to the sequence of the above indicated NCBI Reference Sequence of the PDE4D8 transcript, and also relates to the corresponding amino acid sequence as set forth in SEQ ID NO: 16, which corresponds to the protein sequence defined in NCBI Protein Accession Reference Sequence NP _001184148.1 encoding the PDE4D8 polypeptide.

The term "phosphodiesterase 4D9" or "PDE4D9" relates to the splice variant 9 of the human phosphodiesterase PDE4D, i.e. the human phosphodiesterase PDE4D9 gene, preferably to the sequence as defined in NCBI Reference Sequence: NM_001197220.1, more preferably to the nucleotide sequence as set forth in SEQ ID NO: 17, which corresponds to the sequence of the above indicated NCBI Reference Sequence of the PDE4D9 transcript, and also relates to the corresponding amino acid sequence as set forth in SEQ ID NO: 18, which corresponds to the protein sequence defined in NCBI Protein Accession Reference Sequence NP _001184149.1 encoding the PDE4D9 polypeptide. The term "phosphodiesterase 4D9" or "PDE4D9" also relates to the amplicon that can be generated by the primer pair PDE1D5_forward (SEQ ID NO: 29) and the PDE1D5_reverse (SEQ ID NO: 30) and can be detected by probe SEQ ID NO: 31.

The terms "PDE4D1", "PDE4D2", "PDE4D3", "PDE4D4", "PDE4D5", "PDE4D6", "PDE4D7", "PDE4D8" and "PDE4D9" also comprises nucleotide sequences showing a high degree of homology to PDE4D1, PDE4D2, PDE4D3, PDE4D4, PDE4D5, PDE4D6, PDE4D7, PDE4D8 and PDE4D9 respectively, e.g. nucleic acid sequences being at least 75%, 80%, 85%, 90%, 91%, 92%, 93%, 94%, 95%, 96%, 97%, 98% or 99% identical to the sequence as set forth in SEQ ID NOs: 1, 3, 5, 7, 9, 11, 13, 15 or 17 respectively or amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 respectively or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1, 3, 5, 7, 9, 11, 13, 15 or 17.

The term "expression level" as used herein refers to the amount of PDE4D variant transcript and/or PDE4D protein derivable from a defined number of cells or a defined tissue portion, preferably to the amount of PDE4D variant transcript and/or PDE4D variant protein obtainable in a standard nucleic acid (e.g. RNA) or protein extraction procedure. Suitable extraction methods are known to the person skilled in the art.

When used herein the term "ABCC5" refers to the human Multidrug resistance-associated protein 5 gene (Ensembl: ENSG00000114770), for example, to the sequence as defined in NCBI Reference Sequence NM_001023587.3, specifically, to the nucleotide sequence as set forth in SEQ ID NO:34, which correspond to the sequences of the above indicated NCBI Reference Sequences of the ABCC5 transcript, and also relates to the corresponding amino acid sequence for example as set forth in SEQ ID NO:35, which correspond to the protein sequences defined in NCBI Protein Accession Reference Sequence NP_001018881.1 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being at least 75%, 80%, 85%, 90%, 91%, 92%, 93%, 94%, 95%, 96%, 97%, 98% or 99% identical to the sequence as set forth in SEQ ID NO:35 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:34.

Determining the expression levels of the PDE4D isoforms may be performed using methods known in the art. For example, the gene expression levels may be determined by detecting mRNA expression using one or more primers and/or probes and/or one or more sets thereof. Moreover, the gene expression profile may be determined by an amplification based method and/or microarray analysis and/or RNA sequencing. The determining of the gene expression profile may include performing Real-Time Quantitative Polymerase Chain Reaction (RT-qPCR) on RNA extracted from the biological sample. In other embodiments, the gene expression profile is determined by RNA sequencing, conventional PCR (using, e.g., end point analysis by gel electrophoresis), or multiplex-PCR. In the case of RT-qPCR, the determining of the gene expression profile may include determining a threshold cycle (Ct) value for PDE4D and ABCC5 and optionally each of the one or more reference genes. The PCR may be performed with at least one primer and/or probe for measuring a reference gene selected from HPRT1, TUBA1B, PUM1, and TBP.

Preferably the expression levels, e.g. the PDE4D and ABCC5 expression levels have been or are determined for the prostate cancer patient at the time of taking a biopsy and/or at the time of prostate surgery, such as partial or total removal of the prostate (prostatectomy). Thus ideally the expression levels are determined in a sample obtained during biopsy or prostatectomy. Thus the sample may be taken from the biopsy or resected prostate, but may also be a separate sample as described herein.

When used herein the term "outcome" refers to a prostate cancer related outcome. The outcome may for example refer to time to death, time to cancer related death, time to biochemical recurrence or time to metastasis. The predicted outcome may be expressed as chance that the outcome will occur or not occur. Such predicted chance may be set for a predetermined time span. For example the outcome may be chance of biochemical recurrence within 5 years. In this context a favorable or good outcome or an unfavorable or bad outcome can be

When used herein the method predicts a good outcome or a poor outcome. A good outcome may be a reduced chance of a non-favorable outcome occurring. Non-limiting examples of non-favorable outcomes are: biochemical recurrence, metastasis, cancer specific death or prostate cancer specific death. Alternatively a good outcome may be defined as an increased or high chance of a desirable outcome occurring, for example a 50% chance or more, e.g. 60%, 70%, 80%, or even 90% or more chance of metastasis free survival for at least 100 months, preferably 120 months, 140 months, 160 months, 180 months, or even 200 months or more. It is understood that other clinical end-points may be used as well such as but not limited to post-surgical prostate cancer specific survival, overall survival, post-androgen deprivation therapy prostate cancer specific survival, post salvage radiation therapy prostate cancer specific survival, or post-surgical progression free survival. It is further understood that the term good outcome should be interpreted in the context of the group from which the patient is selected. For example if the patient is from a groups of prostate cancer patients all having a ISUP grade of 1 or 2, their outcome is generally very favorable to begin with, so in this case a good outcome should be viewed in respect to this group (even more favorable). Determining what constitutes a favorable or non-favorable outcome can easily be determined.

When used herein a poor outcome may be an increased chance of a non-favorable outcome occurring. Non-limiting examples of non-favorable outcomes are: biochemical recurrence, metastasis, cancer specific death or prostate cancer specific death. Alternatively a poor outcome may be defined as an decreased or low chance of a desirable outcome occurring, for example a 80% chance or less, e.g. 70%, 60%, 50%, or even 40% or less chance of metastasis free survival for at least 200 months, preferably 180 months, 160 months, 140 months, 120 months, or even 100 months or less. It is understood that other clinical end-points may be used as well such as but not limited to post-surgical prostate cancer specific survival, overall survival, post-androgen deprivation therapy prostate cancer specific survival, post salvage radiation therapy prostate cancer specific survival, or post-surgical progression free survival. It is further understood that the term poor outcome should be interpreted in the context of the group from which the patient is selected.

In an embodiment the outcome is selected from post-surgical metastasis free survival time, post-surgical prostate cancer specific survival, overall survival, post-androgen deprivation therapy prostate cancer specific survival, post salvage radiation therapy prostate cancer specific survival, or post-surgical progression free survival.

In an embodiment the expression levels are used in a PDE4D_ABCC5 score, and the prediction is performed by comparing the PDE4D_ABCC5 score to a predetermined threshold, wherein a score below or equal to the threshold indicates a poor out, and a score above the threshold indicates a good outcome.

When used herein the prediction is based on the PDE4D and ABCC5 expression levels. A simple linear model may be used to combine these expression levels. In an embodiment the expression levels are combined in a PDE4-D_ABCC5 Score. For example, the score may be calculated by subtracting the log2 value of the quantified ABCC5 expression level from the log2 value of the quantified PDE4D expression level. In an embodiment the quantified expression level is transcripts per million (TPM).

In an embodiment the expression levels are used in a PDE4D_ABCC5 score which is calculated as the difference between the PDE4D expression levels and the ABCC5 expression levels, preferably based on transcripts per million, more preferably calculated as PDE4D_ABCC5 score = $2Log(TPM_{PDE4D})$ - $2Log(TPM_{ABCC5})$, wherein $TPM_{PDE4D}$ indicates the transcript per million for PDE4D, preferably obtained from RNA sequencing, and wherein $TPM_{ABCC5}$ indicates the transcript per million for ABCC5, preferably obtained from RNA sequencing.

The percentile ranking procedures first of all ranks all values from smallest to largest (i.e., the patient sample with the smallest PDE4D_ABCC5_Score becomes the first sample in that ranked order and the sample with the largest PDE4D_ABCC5_Score becomes the last sample in that ranked order). Then, the continuous distribution of -2 to +7 is transferred into a distribution between 0 to 100% (or 0 to 1) where the order of the original PDE4D_ABCC5_Scores remain the same as in the original data.

Percentile ranking is a statistical technique used to rank data points within a dataset relative to the other data points in that set. It expresses the relative standing of a value in terms of the percentage of data points that fall below it. The process is commonly used for numeric variables and provides insights into how a specific value compares to the rest of the dataset.

An exemplary way to perform percentile ranking for Numeric Variables start with sorting the data by arranging the numeric dataset in ascending order. This ensures that lower values are ranked lower and higher values are ranked higher. Next ranks are assigned to each data point in the sorted dataset, starting from the smallest value. The smallest value gets a rank of 1, the second smallest gets a rank of 2, and so on. Next the Percentile Rank is calculated by using the formula for percentile ranking:

$$\text{Percentile Rank} = \frac{\text{Rank of the Value} - 1}{\text{Total Number of Data Points} - 1} \times 100$$

The numerator represents how many data points are less than or equal to the given value. Subtracting 1 ensures that the lowest value corresponds to 0% (or the 0th percentile). The denominator represents the total range of ranks. The percentile rank tells the percentage of values in the dataset that are below a given value. For example, if a value has a percentile rank of 75, it means that 75% of the data points are below that value.

In an embodiment a threshold is set for the PDE4D_ABCC5_Score to distinguish between a good and a poor predicted outcome. For example, this is done by the Youden index method. The Youden Index identifies the point on the Receiver Operator Characteristic (ROC) curve that maximizes the sum of sensitivity and specificity, or equivalently, minimizes the misclassification rate when sensitivity and specificity are equally important. This may not be the clinically most optimal relevant cut-off in the end (as in clinical reality either sensitivity of specificity might be more important) but it is a data driven method to determine and initial cut-off.

Accordingly in an embodiment the prediction is based on the percentile ranked scores of the PDE4D_ABCC5 score. In an embodiment the threshold is determined by the Youden index method.

In an embodiment the patient has a ISUP score of 1 or more, preferably 2 or more, more preferably 3 or more, most preferably 4 or 5.

The ISUP (International Society of Urological Pathology) Grade, used here interchangeably with the term ISUP score, is a system at predicting how quickly your cancer might spread. It is done using prostate biopsy samples. The ISUP Grade uses a 1 to 5 grade group system. The higher the grade group, the higher the risk of your cancer being aggressive and spreading rapidly.

ISUP grade 1 corresponds to Gleason scores 2-6 and is defined as having only individual discrete well-formed glands; ISUP grade 2 corresponds to Gleason score 3+4=7 and is defined as having predominantly well-formed glands with lesser component of poorly formed/fused/cribriform glands; ISUP grade 3 corresponds to Gleason score 4+3=7 and is defined as having predominantly poorly formed/fused/cribriform glands with lesser component of well-formed glands; ISUP grade 4 corresponds to Gleason scores 4+4=8, 3+5=8 and 5+3=8 and is defined as having only poorly formed/fused/cribriform glands, or predominantly well-formed glands and lesser component lacking glands (or with necrosis), or predominantly lacking glands (or with necrosis) and lesser component of well-formed glands; and ISUP grade 5 corresponds to Gleason scores 9-10, and is defined as lacking gland formation (or with necrosis) with or without poorly formed/fused/cribriform glands (see also Srigley et al., an Urol Assoc J. 2016 Sep-Oct;10(9-10):339-341, hereby incorporated by reference in its entirety).

The computer implemented according to any one of the preceding claims, wherein the prediction is further based on one or more clinical parameters obtained from the patient selected from pre-surgery plasma PSA levels in the patient, pathology pT stage, and EAU BCR Risk score. In an embodiment the one or more clinical parameters and the PDE4D and ABCC5 expression levels are combined to obtain a risk score using a regression function derived from a population of prostate cancer subjects. In an embodiment the PDE4D and ABCC5 expression levels and the one or more clinical parameter are combined with a logistic regression model. In an embodiment the outcome is based on a score calculated from the PDE4D and ABCC5 expression levels and the one or more clinical parameter. For example the score may be calculated as:

$$SCORE = a * PDE4D\_ABCC5\_Score + b * Clinical\_parameter + c$$

wherein each of a, b and c are constants. The PDE4D_ABCC5_Score may for example be calculated as:

$$PDE4D\_ABCC5\_Score = d * [PDE4D] + e * [ABCC5] + f$$

wherein [PDE4D] is a numerical value representing the PDE4D expression level (e.g. normalized expression or transcripts per million) and [ABCC5] is a numerical value representing the ABCC5 expression level.

The method may further be combined with other predictive or prognostic methods to further improve accuracy, for example but not limited to mxCCP, mx GPS, or GC.

When used herein the term mxGPS refers to the MDxHealth genomic prostate cancer score, a commercially available genomic assay designed for men with low-, intermediate-, and high-risk localized prostate cancer to help guide treatment decisions at the time of diagnosis. When used herein mxCCP refers to cell cycle progression score, also referred to as cell cycle prostate score, a commercial test for evaluating prostate cancer progression risk. When used herein GC refers to the Decipher Genomic Classifier score, a commercially available test for prognosticating an outcome in prostate cancer.

It is further preferred that the method comprises:

normalizing the gene expression profile with respect to one or more reference genes selected from the group consisting of: hypoxanthine phosphoribosyltransferase 1 (HPRT1), Tubulin-Alpha-1b (TUBA1B), pumilio RNA-Binding Family Member (PUM1), and TATA box binding protein (TBP), wherein the expression based score is determined based on the normalized gene expression profile.

By normalizing the gene expression profile with respect to one or more reference genes and by determining the expression based score is determined based on the normalized gene expression profile, variability in the determination of the expression based risk score can be reduced. This enables differentiation between real variations in gene expression profiles and variations due to the measurement processes. In this respect, it has been found that HPRT1, TUBA1B, PUM1, and TBP are particularly well suited as reference genes for normalizing the PDE4D or ABCC5 gene expression profile.

Other reference genes which may be additionally or alternatively used for normalizing the PDE4D7 gene expression profile include: actin, beta, mRNA (ACTB); 60S acidic ribosomal phosphoprotein P0 mRNA (RPLP0); Polymerase (RNA) II (DNA Directed) Polypeptide A, 220kDa (POLR2A); Beta-2-Microglobulin (B2M); and Aminolevulinate-Delta-Synthase (ALAS-1).

In an embodiment the method further comprises providing a treatment recommendation based on the prediction. In an embodiment the treatment recommendation when the patient has a good prognosis is:

for subjects with negative pelvic lymph nodes is selected from:

radiation therapy, preferably external beam radiation therapy (EBRT), supplemented with androgen deprivation therapy ADT,
for subjects with positive pelvic lymph nodes the treatment is selected from:

radiation therapy, preferably EBRT, supplemented with ADT and abiraterone; and wherein the treatment recommendation when patient has a poor prognosis is:
the treatment is selected from
radiation therapy, preferably ERBT, supplemented with ADT and one or more of Abiraterone, Apalutamide or Enzalutamide; or
ADT supplemented with taxane based chemotherapy, preferably docetaxel, and supplemented with one of Abiraterone, Apalutamide or Enzalutamide; or
one or more treatments selected from:

PARP inhibitors, preferably Olaparib, Rucaparib, or Niraparib), in case the subject has mutations and/or variants in DNA repair genes or ERG gene translocations or TP53 mutations or loss;
kinase inhibitors, preferably Gefitinib, Capivasertib, or Sorafenib) in case the subject has mutations in protein kinases;
mTOR inhibitors, preferably Everolimus in case the subject has PTEN loss or PIK3CA mutations; or
Immune Checkpoint inhibitors, preferably Pembroluzimab or Nivolumab in case the subject has high tumor mutational burden (TMB);

wherein said treatment is optionally further comprises ADT and/or radiotherapy, preferably EBRT.

In a preferred embodiment the ADT is selected from; Luteinizing hormone-releasing hormone (LHRH) agonist alone, LHRH agonists combined with a first or second generation antiandrogen, or LHRH antagonists. In an embodiment the LHRH agonist is selected from Goserelin, Leuprolide, or Triptoreli. In an embodiment the first generation antiandrogen is selected from Nilutamide, Flutamide, or Bicalutamide, In an embodiment the second generation antiandrogen is selected from Apalutamide, Darolutamide, Enzalutamide, and Proxalutamide. In an embodiment the LHRH antagonist is selected from Degarelix or Relugolix.

Androgen deprivation therapy also called androgen ablation therapy or androgen suppression therapy, is an antihormone therapy whose main use is in treating prostate cancer. ADT reduces the levels of androgen hormones, with drugs or surgery, to prevent the prostate cancer cells from growing. Surgical based ADT includes orchiectomy (surgical castration by removing of the testicles). Drug based ADT can be chemical castration or antiandrogen therapy. Chemical castration can be performed by LHRH agonists and antagonists, which both lower the amount of testosterone made by the testicles. Antiandrogen therapy can use steroidal or non-steroidal antiandrogens. Thus when referred herein to ADT the therapy may be one or more selected from orchiectomy, chemical castration and antiandrogen therapy. Preferably the ADT refers to antiandrogen therapy, more preferably to non-steroidal antiandrogen therapy.

Non limiting examples of LHRH agonists, also referred to as GnRH agonists, are Azagly-nafarelin, Buserelin, Deslorelin, Fertirelin, Gonadorelin, Goserelin, Histrelin, Lecirelin, Leuprorelin, Nafarelin, Peforelin, and Triptorelin. Thus in an embodiment the LHRH agonist (GnRH agonist) is selected from Azagly-nafarelin, Buserelin, Deslorelin, Fertirelin, Gonadorelin, Goserelin, Histrelin, Lecirelin, Leuprorelin, Nafarelin, Peforelin, and Triptorelin.

Non limiting examples of steroidal antiandrogens are Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone. Thus in an embodiment the steroidal antiandrogen is selected from Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone.

Non limiting examples of non-steroidal androgen receptor inhibitor are Flutamide, Nilutamide, Bicalutamide, Enzalutamide, Clascoterone, Apalutamide, Proxalutamide, Cimetidine, Topilutamide, and Darolutamide. Thus in an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide.

Androgen deprivation therapy may be provided as adjuvant androgen deprivation therapy, neoadjuvant androgen deprivation therapy, or salvage androgen deprivation therapy, among others. Thus in an embodiment the ADT is provided as adjuvant androgen deprivation therapy, neoadjuvant androgen deprivation therapy, or salvage androgen deprivation therapy. In a particularly preferred embodiment the ADT is neoadjuvant androgen deprivation therapy or adjuvant androgen deprivation therapy, more preferably neoadjuvant androgen deprivation therapy.

The term adjuvant androgen deprivation therapy (AADT) when used herein refers to the provision of androgen deprivation therapy after surgery (e.g. partial or complete removal of the prostate) or radiotherapy.

Neoadjuvant androgen deprivation therapy (NADT) is a recommended treatment for patients diagnosed with localized prostate cancer. NADT is systemic therapy administered after the diagnosis of prostate cancer but before locoregional therapy such as radical prostatectomy (RP) or radiation. With NADT prior to RP, the intent is to eradicate malignant androgen-dependent cells, in the hope that sufficient tumor regression will permit complete resection of residual prostate cancer, improving pathologic outcome and survival. The role of preoperative androgen deprivation remains controversial, however. The present methods, products, and uses aim to identify those patients for whom NADT likely is beneficial, and to identify those patients whom are more likely to not benefit form NADT and thus should receive an alternative or additional treatment.

The term salvage androgen deprivation therapy (SADT) when used herein refers to the use of ADT to treat biochemical recurrence, metastasis or tumor growth after treatment of prostate cancer, typically by surgery or radiation. Biochemical recurrence (BCR) indicates a rise of prostate-specific antigen (PSA) levels in the blood of a prostate cancer patient after treatment with surgery or radiation. Typically SADT is performed well after (e.g. one year or more) after initial treatment of the prostate cancer by for example surgery or radiation. Thus SADT is used to treat relapse of prostate cancer.

Thus when used herein the term ADT may refer to NADT, SADT or AADT.

When used herein the term chemotherapy refers to a chemotherapeutic agents, also known as cytotoxic agents or cytostatic drugs, that are known to be of use in chemotherapy for cancer, and have the intention to kill tumor cells or reduce tumor size. Non limiting examples are Alkylating agents such as Altretamine, Bendamustine, Busulfan, Carboquone, Carmustine, Chlorambucil, Chlormethine, Chlorozotocin, Cyclophosphamide, Dacarbazine, Fotemustine, Ifosfamide, Lomustine, Melphalan, Melphalan flufenamide, Mitobronitol, Nimustine, Nitrosoureas, Pipobroman, Ranimustine, Semustine, Streptozotocin, Temozolomide, Thiotepa, Treosulfan, Triaziquone, Triethylenemelamine, Trofosfamide, and Uramustine; or Anthracyclines such as Aclarubicin, Daunorubicin, Doxorubicin, Epirubicin, Idarubicin, Mitoxantrone, Pirarubicin, Valrubicin, and Zorubicin; or Cytoskeletal disruptors (taxanes) such as Abraxane, Cabazitaxel, Docetaxel, Larotaxel, Paclitaxel, Taxotere, and Tesetaxel; or Epothilones such as Ixabepilone; or Histone deacetylase inhibitors such as Entinostat, Romidepsin, Vorinostat, and Zabadinostat; or Inhibitors of topoisomerase I such as Belotecan, Camptothecin, Exatecan, Gimatecan, Irinotecan, and Topotecan; or Inhibitors of topoisomerase II such as Etoposide, Teniposide, and Tafluposide; or Kinase inhibitors such as Bortezomib, Erlotinib, Gefitinib, Imatinib, Vemurafenib, and Vismodegib; or Nucleotide analogs and precursor analogs such as Azacitidine, Azathioprine, Capecitabine, Cladribine, Clofarabine, Cytarabine, Decitabine, Doxifluridine, Fludarabine, Fluorouracil, Gemcitabine, Hydroxyurea, Mercaptopurine, Methotrexate, Nelarabine, Pemetrexed, and Tioguanine (formerly Thioguanine); or Peptide antibiotics such as Actinomycin, and Bleomycin; or Platinum-based agents such as Main Carboplatin, Cisplatin, Dicycloplatin, Oxaliplatin, Nedaplatin, and Satraplatin; or Retinoids such as Alitretinoin, Bexarotene, and Tretinoin; or Vinca alkaloids and derivatives such as Vinblastine, Vincristine, Vindesine, and Vinorelbine.

When used herein targeted therapy may refer to targeted radionuclide therapy. Targeted radionuclide therapy refers to radioisotopes bound to or incorporated in a targeting agent such as PSMA or an antibody such as a nanobody. A non-limiting example suitable for prostate cancer is PSMA Lutetium-177. Targeted therapy may also refer to a therapy that acts on a protein or pathway particularly re;lied on by cancer cells. For example a targeted therapy for prostate cancer may target PARP (PARP inhibitors). Non limiting examples of PARP inhibitors are Rucaparib, Olaparib, Talazoparib, and Niraparib plus abiraterone.

Non limiting examples of immunotherapy are cancer vaccine (such as but not limited to Sipuleucel-T (Provenge) and prostatic acid phosphatase (PAP)), or Immune checkpoint inhibitors, such as but not limited to Ipilimumab, Nivolumab, Pembrolizumab, Cemiplimab, Spartalizumab, Camrelizumab, Sintilimab, Tislelizumab, Toripalimab, Dostarlimab, Retifanlimab, AMP-224, MEDI0680, Atezolizumab, Avelumab, Durvalumab, Envafolimab, Cosibelimab, AUNP-12, CA-170, anti-OX40 antibody BMS 986178, Tremelimumab, Abatacept, Belatacept, Sotorasib, Adagrasib, Relatlimab, Lisavanbulin, PHI-101, and Quemliclustat.

In a second aspect the invention relates to a computer program product comprising instructions which, when the program is executed by a computer, cause the computer to carry out a method for predicting the outcome for a subject suffering from prostate cancer, based on the PDE4D and ABCC5 expression levels determined in a prostate cancer sample obtained from the subject, the method comprising: predicting the outcome for the subject based on the PDE4D and ABCC5 expression levels.

In a further aspect the invention relates to a kit of parts comprising primers and/or probes for specifically detecting and quantifying the expression level in biological sample of the genes PDE4D, and ABCC5, preferably wherein the kit is a kit selected from an RNA sequencing kit, a PCR kit, a qPCR kit, a digital PCR kit, an immunohistochemistry kit, an ELISA kit, and an in situ RNA hybridization kit. In an embodiment the kit is for selectively amplifying PDE4D, and ABCC5 mRNA in a sample. In an embodiment the kit is for detecting PDE4D, and ABCC5 protein in a sample. In an embodiment the kit is for quantifying PDE4D, and ABCC5 mRNA or protein. In an embodiment the kit is suitable for use in a method for determining an outcome for a subject having prostate cancer.

Use of a kit to determine an outcome for a subject suffering from prostate cancer, the use comprising using the kit to determine the expression levels of PDE4D and ABCC5 in a prostate cancer sample obtained from the subject, and determining the outcome for the subject based on the determined expression levels, wherein the kit comprises means for determining the expression levels of PDE4D and ABCC5 in a biological sample. In an embodiment the kit is the kit as broadly described herein above.

In a fourth aspect the invention relates to a therapy for use in the treatment of prostate cancer in a subject, the use comprising receiving the result of the determined expression levels of PDE4D and ABCC5 in a prostate cancer sample obtained from the subject, predicting the outcome for the subject based on the PDE4D and ABCC5 expression levels, wherein the outcome is a good outcome or a poor outcome, and administering the therapy when a poor outcome is predicted,
wherein the therapy is selected from:

radiation therapy, preferably ERBT, supplemented with ADT and one or more of Abiraterone, Apalutamide or Enzalutamide; or
ADT supplemented with taxane based chemotherapy, preferably docetaxel, and supplemented with one of Abiraterone, Apalutamide or Enzalutamide; or
one or more treatments selected from:

PARP inhibitors, preferably Olaparib, Rucaparib, or Niraparib), in case the subject has mutations and/or variants in DNA repair genes or ERG gene translocations or TP53 mutations or loss;
kinase inhibitors, preferably Gefitinib, Capivasertib, or Sorafenib) in case the subject has mutations in protein kinases;
mTOR inhibitors, preferably Everolimus in case the subject has PTEN loss or PIK3CA mutations; or
Immune Checkpoint inhibitors, preferably Pembroluzimab or Nivolumab in case the subject has high tumor mutational burden (TMB).

In an embodiment the treatment further comprises ADT and/or radiotherapy, preferably EBRT. In an embodiment the ADT is selected from; Luteinizing hormone-releasing hormone (LHRH) agonist alone, LHRH agonists combined with a first or second generation antiandrogen, or LHRH antagonists. In an embodiment the LHRH agonist is selected from Goserelin, Leuprolide, or Triptorelin. In an embodiment the first generation antiandrogen is selected from Nilutamide, Flutamide, or Bicalutamide. In an embodiment the second generation antiandrogen is selected from Apalutamide, Darolutamide, Enzalutamide, and Proxalutamide. In an embodiment the LHRH antagonist is selected from Degarelix or Relugolix.

In a further aspect the invention relates to a method of treating prostate cancer in a subject in need thereof, the method comprising:

receiving the result of the determined expression levels of PDE4D and ABCC5 in a prostate cancer sample obtained from the subject, predicting the outcome for the subject based on the PDE4D and ABCC5 expression levels, wherein the outcome is a good outcome or a poor outcome, and administering a therapy when a poor outcome is predicted, wherein the therapy is selected from:

radiation therapy, preferably ERBT, supplemented with ADT and one or more of Abiraterone, Apalutamide or Enzalutamide; or
ADT supplemented with taxane based chemotherapy, preferably docetaxel, and supplemented with one of Abiraterone, Apalutamide or Enzalutamide; or
one or more treatments selected from:

PARP inhibitors, preferably Olaparib, Rucaparib, or Niraparib), in case the subject has mutations and/or variants in DNA repair genes or ERG gene translocations or TP53 mutations or loss;
kinase inhibitors, preferably Gefitinib, Capivasertib, or Sorafenib) in case the subject has mutations in protein kinases;
mTOR inhibitors, preferably Everolimus in case the subject has PTEN loss or PIK3CA mutations; or
Immune Checkpoint inhibitors, preferably Pembroluzimab or Nivolumab in case the subject has high tumor mutational burden (TMB);
or administering a therapy when the patient has a good prognosis, wherein the therapy is selected from:

for subjects with negative pelvic lymph nodes is selected from: radiation therapy, preferably external beam radiation therapy (EBRT), supplemented with androgen deprivation therapy ADT,
for subjects with positive pelvic lymph nodes the treatment is selected from: radiation therapy, preferably EBRT, supplemented with ADT and abiraterone;

In an embodiment the prostate cancer sample is a resected prostate, a tumor biopsy, a prostate biopsy, a tissue resection, or any other sample obtained from the patient comprising at least one prostate cancer cell. In an embodiment the prostate cancer sample is obtained prior to the start of the therapy.

When used herein the term subject refers to a human or a non-human animal such as a mammal. When used herein the subject is a subject suffering from prostate cancer. The subject may be a patient, hence the terms subject and patient are used interchangeably herein. Preferably the subject has undergone or will undergo surgery. The surgery is preferably partial or complete removal of the prostate (radical prostatectomy), but the surgery may also be radiation therapy. In an embodiment the subject the subject has a pre-operative PSA level of 1 ng/ml or more, preferably 2, 3, 4,5, 6, 7, 8, 9, 10, 11, 12, 13, 14, 15, 16, 17, 18, 19or even 20 ng/ml or more. In an embodiment the subject has an ISUP score between 1-5, preferably 2-5, more preferably 3-5, most preferably 4 or 5. In an embodiment the subject has an ISUP score of 1, 2, 3, 4, or 5. In embodiment the subject has a pathology stage of pT2, pT3, pT3a, pT3b, pT3c, or pT4, preferably pT3, pT3a, pT3b, pT3c, or pT4, more preferably pT3b, pT3c, or pT4, most preferably pT3c, or pT4.

The terms outcome, good outcome and poor outcome are defined herein above.

In a preferred embodiment the ADT is selected from; Luteinizing hormone-releasing hormone (LHRH) agonist alone, LHRH agonists combined with a first or second generation antiandrogen, or LHRH antagonists. In an embodiment the LHRH agonist is selected from Goserelin, Leuprolide, or Triptoreli. In an embodiment the first generation antiandrogen is selected from Nilutamide, Flutamide, or Bicalutamide, In an embodiment the second generation antiandrogen is selected from Apalutamide, Darolutamide, Enzalutamide, and Proxalutamide. In an embodiment the LHRH antagonist is selected from Degarelix or Relugolix.

Androgen deprivation therapy also called androgen ablation therapy or androgen suppression therapy, is an antihormone therapy whose main use is in treating prostate cancer. ADT reduces the levels of androgen hormones, with drugs or surgery, to prevent the prostate cancer cells from growing. Surgical based ADT includes orchiectomy (surgical castration by removing of the testicles). Drug based ADT can be chemical castration or antiandrogen therapy. Chemical castration can be performed by LHRH agonists and antagonists, which both lower the amount of testosterone made by the testicles. Antiandrogen therapy can use steroidal or non-steroidal antiandrogens. Thus when referred herein to ADT the therapy may be one or more selected from orchiectomy, chemical castration and antiandrogen therapy. Preferably the ADT refers to antiandrogen therapy, more preferably to non-steroidal antiandrogen therapy.

Non limiting examples of LHRH agonists, also referred to as GnRH agonists, are Azagly-nafarelin, Buserelin, Deslorelin, Fertirelin, Gonadorelin, Goserelin, Histrelin, Lecirelin, Leuprorelin, Nafarelin, Peforelin, and Triptorelin. Thus in an embodiment the LHRH agonist (GnRH agonist) is selected from Azagly-nafarelin, Buserelin, Deslorelin, Fertirelin, Gonadorelin, Goserelin, Histrelin, Lecirelin, Leuprorelin, Nafarelin, Peforelin, and Triptorelin.

Non limiting examples of steroidal antiandrogens are Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone. Thus in an embodiment the steroidal antiandrogen is selected from Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone.

Non limiting examples of non-steroidal androgen receptor inhibitor are Flutamide, Nilutamide, Bicalutamide, Enzalutamide, Clascoterone, Apalutamide, Proxalutamide, Cimetidine, Topilutamide, and Darolutamide. Thus in an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide.

Androgen deprivation therapy may be provided as adjuvant androgen deprivation therapy, neoadjuvant androgen deprivation therapy, or salvage androgen deprivation therapy, among others. Thus in an embodiment the ADT is provided as adjuvant androgen deprivation therapy, neoadjuvant androgen deprivation therapy, or salvage androgen deprivation therapy. In a particularly preferred embodiment the ADT is neoadjuvant androgen deprivation therapy or adjuvant androgen deprivation therapy, more preferably neoadjuvant androgen deprivation therapy.

Thus when used herein the term ADT may refer to NADT, SADT or AADT.

When used herein the term chemotherapy refers to a chemotherapeutic agents, also known as cytotoxic agents or cytostatic drugs, that are known to be of use in chemotherapy for cancer, and have the intention to kill tumor cells or reduce tumor size. Non limiting examples are Alkylating agents such as Altretamine, Bendamustine, Busulfan, Carboquone, Carmustine, Chlorambucil, Chlormethine, Chlorozotocin, Cyclophosphamide, Dacarbazine, Fotemustine, Ifosfamide, Lomustine, Melphalan, Melphalan flufenamide, Mitobronitol, Nimustine, Nitrosoureas, Pipobroman, Ranimustine, Semustine, Streptozotocin, Temozolomide, Thiotepa, Treosulfan, Triaziquone, Triethylenemelamine, Trofosfamide, and Uramustine; or Anthracyclines such as Aclarubicin, Daunorubicin, Doxorubicin, Epirubicin, Idarubicin, Mitoxantrone, Pirarubicin, Valrubicin, and Zorubicin; or Cytoskeletal disruptors (taxanes) such as Abraxane, Cabazitaxel, Docetaxel, Larotaxel, Paclitaxel, Taxotere, and Tesetaxel; or Epothilones such as Ixabepilone; or Histone deacetylase inhibitors such as Entinostat, Romidepsin, Vorinostat, and Zabadinostat; or Inhibitors of topoisomerase I such as Belotecan, Camptothecin, Exatecan, Gimatecan, Irinotecan, and Topotecan; or Inhibitors of topoisomerase II such as Etoposide, Teniposide, and Tafluposide; or Kinase inhibitors such as Bortezomib, Erlotinib, Gefitinib, Imatinib, Vemurafenib, and Vismodegib; or Nucleotide analogs and precursor analogs such as Azacitidine, Azathioprine, Capecitabine, Cladribine, Clofarabine, Cytarabine, Decitabine, Doxifluridine, Fludarabine, Fluorouracil, Gemcitabine, Hydroxyurea, Mercaptopurine, Methotrexate, Nelarabine, Pemetrexed, and Tioguanine (formerly Thioguanine); or Peptide antibiotics such as Actinomycin, and Bleomycin; or Platinum-based agents such as Main Carboplatin, Cisplatin, Dicycloplatin, Oxaliplatin, Nedaplatin, and Satraplatin; or Retinoids such as Alitretinoin, Bexarotene, and Tretinoin; or Vinca alkaloids and derivatives such as Vinblastine, Vincristine, Vindesine, and Vinorelbine.

When used herein targeted therapy may refer to targeted radionuclide therapy. Targeted radionuclide therapy refers to radioisotopes bound to or incorporated in a targeting agent such as PSMA or an antibody such as a nanobody. A non-limiting example suitable for prostate cancer is PSMA Lutetium-177. Targeted therapy may also refer to a therapy that acts on a protein or pathway particularly re;lied on by cancer cells. For example a targeted therapy for prostate cancer may target PARP (PARP inhibitors). Non limiting examples of PARP inhibitors are Rucaparib, Olaparib, Talazoparib, and Niraparib plus abiraterone.

Non limiting examples of immunotherapy are cancer vaccine (such as but not limited to Sipuleucel-T (Provenge) and prostatic acid phosphatase (PAP)), or Immune checkpoint inhibitors, such as but not limited to Ipilimumab, Nivolumab, Pembrolizumab, Cemiplimab, Spartalizumab, Camrelizumab, Sintilimab, Tislelizumab, Toripalimab, Dostarlimab, Retifanlimab, AMP-224, MED10680, Atezolizumab, Avelumab, Durvalumab, Envafolimab, Cosibelimab, AUNP-12, CA-170, anti-OX40 antibody BMS 986178, Tremelimumab, Abatacept, Belatacept, Sotorasib, Adagrasib, Relatlimab, Lisavanbulin, PHI-101, and Quemliclustat.

In a further aspect of the invention, the method is used to determine effectiveness of ADT such as NADT, AADT, or SADT, or salvage radiotherapy (SRT). Thus in an embodiment the invention relates to a method of predicting the outcome of a therapy for treating prostate cancer, the method comprising receiving the result of the determined expression levels of PDE4D and ABCC5 in a prostate cancer sample obtained from the subject, and predicting the outcome of the therapy for the subject based on the PDE4D and ABCC5 expression levels, wherein the outcome is a good outcome or a poor outcome, and wherein the therapy is ADT, preferably AADT, NADT or SADT, or wherein the therapy is radiotyion therapy, preferably SRT.

Accordingly, in a further aspect the invention relates to a therapy for use in the treatment of prostate cancer in a subject, the use comprising receiving the result of the determined expression levels of PDE4D and ABCC5 in a prostate cancer sample obtained from the subject, predicting the outcome of the therapy for the subject based on the PDE4D and ABCC5 expression levels, wherein the outcome is a good outcome or a poor outcome, and administering the therapy when a good outcome is predicted. In an embodiment when a poor outcome is predicted an alternative therapy is provided.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

Examples

Having now generally described the invention, the same will be more readily understood through reference to the following examples which is provided by way of illustration and is not intended to be limiting of the present invention.

**Example 1** - **construction of PDE4D-ABCC5 based model**

A PDE4D_ABCC5_Score was defined as PDE4D(log2) - ABCC5(log2); the Score uses the TPM (transcript per million) gene expression values for PDE4D and ABCC5 as determined by the RNAseq data processing. Then the log2 values from the TPM of the two genes are calculated and the ABCC5(log2) is subtracted from the PDE4D(log2). The resulting number is the PDE4D_ABCC5_Score;

To morte easily allow comparison of the PDE4D_ABCC5_Score a percentile ranked scores of the PDE4-D_ABCC5 Score is calculated by sorting the data by arranging the numeric dataset in ascending order. Next ranks are assigned to each data point in the sorted dataset, starting from the smallest value. The smallest value gets a rank of 1, the second smallest gets a rank of 2, and so on. Next the Percentile Rank is calculated by using the formula for percentile ranking:

$$\text{Percentile Rank} = \frac{\text{Rank of the Value} - 1}{\text{Total Number of Data Points} - 1} \times 100$$

The numerator represents how many data points are less than or equal to the given value. Subtracting 1 ensures that the lowest value corresponds to 0% (or the 0th percentile). The denominator represents the total range of ranks. The percentile rank tells the percentage of values in the dataset that are below a given value. For example, if a value has a percentile rank of 75, it means that 75% of the data points are below that value.

Next a threshold was calculated using the Youden index method. The Youden Index identifies the point on the ROC curve that maximizes the sum of sensitivity and specificity, or equivalently, minimizes the misclassification rate when sensitivity and specificity are equally important.

The threshold is used to distinguish between poor and good outcome prediction in the patient cohort.

**Example 2** - **testing of the PDE4D-ABCC5 model on patient data**

For testing the following cohorts were used:

**Patient cohort #1**: 653 prostate cancer patients operated 2000-2010 with around >60% ISUP 1 and 2 and <40% ISUP 3-5; 10+ years follow-up and outcomes available (SRT, ADT, BCR, MFS, PCSM, ACM); tissue used for qPCR analysis: RP biopsy punch; RNAseq data available for 571 patients

**Patient cohort #2**: 402 prostate cancer patients operated 1995-2010 with around 30-40% ISUP 1 and 2 and 50-60% ISUP 3-5; 10+ years follow-up and outcomes available (SRT, ADT, BCR, MFS, PCSM, ACM); tissue used for qPCR analysis: RP biopsy punch; RNAseq data available for 153 patients

**Patient cohort #3**: 151 prostate cancer patients operated 1995-2010 with around 30-40% ISUP 1 and 2 and 50-60% ISUP 3-5; 10+ years follow-up and outcomes available (SRT, ADT, BCR, MFS, PCSM, ACM); tissue used for qPCR analysis: RP biopsy punch; RNAseq data available for 146 patients

**Patient cohort #4**: 178 prostate cancer patients operated 2000-2010 with around >60% ISUP 1 and 2 and <40% ISUP 3-5; 10+ years follow-up and outcomes available (SRT, ADT, BCR, MFS, PCSM, ACM); tissue used for qPCR analysis: diagnostic needle biopsy punch; RNAseq data available for 171 patients

First the model was tested with the clinical endpoint post-surgical metastasis free survival. The pPDE4-D_ABCC5_Score and threshold was calculated as described above and applied in all subsequent analyses. Fig. 1 shows Kaplan Meier curves for all ISUP scores (1-5), Fig. 2 for ISUP score 2-5 and Fig. 3 for ISUP scores 3-5.

Next disease specific survival was used as clinical endpoint. Fig. 4 shows Kaplan Meier curves for all ISUP scores (1-5), Fig. 5 for ISUP score 2-5, Fig. 6 for ISUP scores 3-5, and Fig. 7 for ISUP scores 4 or 5.

Next post-surgical disease specific survival was used as clinical endpoint and the patients were separated based on pre-operative PSA levels. Fig. 8 shows Kaplan Meier curves for patients having pre-operative PSA levels of below or equal to 10 ng/ml, Fig. 9 for patients having pre-operative PSA levels of 10-20 ng/ml and Fig. 10 for patients having pre-operative PSA levels above 20 ng/ml.

Next post-surgical disease specific survival was used as clinical endpoint and the patients were separated based on pathology stage. Fig. 11 shows Kaplan Meier curves for patients having pathology stage pT2, Fig. 12 for patients having pathology stage pT3, Fig. 13 for patients having pathology stage pT3a, Fig. 14 for patients having pathology stage pT3b, Fig. 15 for patients having pathology stage pT3b, pT3c, or pT4.

Next post-surgical disease specific survival was used as clinical endpoint and the patients were separated based on ISUP score. Fig. 16 shows Kaplan Meier curves for patients having an ISUP score of 2, 3, 4, or 5, Fig. 17 shows for patients with ISUP score 3, 4, or 5.

Next post-surgical prostate cancer specific survival was used as an endpoint to construct an ROC curve for the percentile ranked scores of the PDE4D_ABCC5 Score in patients having an ISUP score of 2-5. Indicated is the determined threshold based on optima sensitivity vs specificity (Fig. 18). In Fig. 19 the ROC curve for the PDE4D-ABCC5 model is compared other predictive models (mxCCP, mxGPS and Genomic Classifier (GC), see e.g. Salami et al., JCI Insight. 2018 Nov 2;3(21):e123468, hereby incorporated by reference in its entirety). To allow comparison a percentile ranking was also created for the reference scores. It can be seen that the PDE4D_ABCC5 model has a higher area under the curve compared to reference models and thus provides an improved performance.

Next post-surgical disease specific survival was used as clinical endpoint and the patients were separated based on pathology stage, Fig. 20 shows Kaplan Meier curves for patients having pathology stage pT2, pT3a, pT3b, or pT3c/pT4, Fig.21 shows pT3a, pT3b, or pT3c/pT4. As can be seen from the figures the pathology stage poorly distinguishes between outcome and thus is a poor predictor for such.

Next post-surgical disease specific survival was used as clinical endpoint and the patients were separated based on ISUP score and EAU BCR Risk (data not shown). The EAU BCR Risk (high or low) was combined with the pDE4D_ABCC5_Score (high or low) to further classify patients. Fig.22 shows these data for ISUP scores 1-5, Fig. 23 for ISUP 2-5, Fig. 24 for ISUP scores 3-5.

Next post-surgical disease specific survival was used as clinical endpoint for either EAU BCR low risk (Fig. 25) or high risk (Fig. 26) in patients with ISUP score of 2-5. The Kaplan-Meier curve shows difference between pPDE4-D_ABCC5_Score (high or low). The data shows that particularly in the EAU BCR high risk group the pPDE4-D_ABCC5_Score can distinguish between poor and good outcome patients.

Next post-surgical disease specific survival was used as clinical endpoint for either GC low risk (Fig. 27) or high risk (Fig. 28) in patients with ISUP score of 2-5. The Kaplan-Meier curve shows difference between pPDE4-D_ABCC5_Score (high or low). The data shows that particularly in the GC high risk group the pPDE4D_ABCC5_Score can distinguish between poor and good outcome patients.

Next post-surgical disease specific survival was used as clinical endpoint for either mxGPS low risk (Fig. 29) or high risk (Fig. 30) in patients with ISUP score of 2-5. The Kaplan-Meier curve shows difference between pPDE4-D_ABCC5_Score (high or low). The data shows that particularly in both the mxGPS high and low risk group the pPDE4D_ABCC5_Score can distinguish between poor and good outcome patients.

Next post-surgical disease specific survival was used as clinical endpoint for either mxCCP low risk (Fig. 31) or high risk (Fig. 32) in patients with ISUP score of 2-5. The Kaplan-Meier curve shows difference between pPDE4-D_ABCC5_Score (high or low). The data shows that particularly in the mxCCP high risk group the pPDE4-D_ABCC5_Score can distinguish between poor and good outcome patients.

Next post-surgical disease specific survival (Fig. 33-35), post-surgical overall survival (Fig. 36) post-ADT disease specific survival (Fig. 37, 38, 40), or post-SRT disease specific survival (Fig. 39) were used as clinical endpoint in patients groups separated based on ISUP score and combination of pPDE4D_ABCC5_Score and EAU BCR score.

**Claims**

1.  A computer implemented method for predicting an outcome for a subject suffering from prostate cancer, the method comprising:

    receiving the result of the determined expression levels of PDE4D and ABCC5 in a prostate cancer sample obtained from the subject, and
    predicting the outcome for the subject based on the PDE4D and ABCC5 expression levels, wherein the outcome is a good outcome or a poor outcome.

2.  The computer implemented according to claim 1, wherein the expression levels are used in a PDE4D_ABCC5 score, and the prediction is performed by comparing the PDE4D_ABCC5 score to a predetermined threshold, wherein a score below or equal to the threshold indicates a poor outcome, and a score above the threshold indicates a good outcome.

3.  The computer implemented according to claim 1 or 2, wherein the outcome is selected from post-surgical metastasis free survival, post-surgical prostate cancer specific survival, overall survival, post-androgen deprivation therapy prostate cancer specific survival, post salvage radiation therapy prostate cancer specific survival, or post-surgical progression free survival.

4.  The computer implemented according to any one of the preceding claims, wherein the prostate cancer sample is a resected prostate, a tumor biopsy, a prostate biopsy, a tissue resection, or any other sample obtained from the patient comprising at least one prostate cancer cell.

5.  The computer implemented according to any one of the preceding claims, wherein the patient has a ISUP score of 1 or more, preferably 2 or more, more preferably 3 or more, most preferably 4 or 5.

6.  The computer implemented according to any one of the preceding claims, wherein the expression levels are used in a PDE4D_ABCC5 score which is calculated as the difference between the PDE4D expression levels and the ABCC5 expression levels, preferably based on transcripts per million, more preferably calculated as PDE4D_ABCC5 score = $2\text{Log}(\text{TPM}_{PDE4D}) - 2\text{Log}(\text{TPM}_{ABCC5})$, wherein $\text{TPM}_{PDE4D}$ indicates the transcript per million for PDE4D, preferably obtained from RNA sequencing, and wherein $\text{TPM}_{ABCC5}$ indicates the transcript per million for ABCC5, preferably obtained from RNA sequencing.

7.  The computer implemented according to claim 6, wherein the prediction is based on the percentile ranked scores of the PDE4D_ABCC5 score.

8.  The computer implemented according to any one of the preceding claims, wherein the threshold is determined by the Youden index method.

9.  The computer implemented according to any one of the preceding claims, wherein the prediction is further based on one or more clinical parameters obtained from the patient selected from pre-surgery plasma PSA levels in the patient, pathology pT stage, and EAU BCR Risk score.

10. The computer implemented according to claim 9, wherein the one or more clinical parameters and the PDE4D and ABCC5 expression levels are combined to obtain a risk score using a regression function derived from a population of prostate cancer subjects.

11. The computer implemented according to any one of the preceding claims, wherein the method further comprises providing a treatment recommendation based on the prediction.

12. The computer implemented according to claim 11, wherein the treatment recommendation when the patient has a good prognosis is:
    for subjects with negative pelvic lymph nodes is selected from:

    radiation therapy, preferably external beam radiation therapy (EBRT), supplemented with androgen deprivation therapy ADT,
    for subjects with positive pelvic lymph nodes the treatment is selected from:

radiation therapy, preferably EBRT, supplemented with ADT and abiraterone; and wherein the treatment recommendation when patient has a poor prognosis is:

the treatment is selected from

radiation therapy, preferably ERBT, supplemented with ADT and one or more of Abiraterone, Apalutamide or Enzalutamide; or

ADT supplemented with taxane based chemotherapy, preferably docetaxel, and supplemented with one of Abiraterone, Apalutamide or Enzalutamide; or

one or more treatments selected from:

> PARP inhibitors, preferably Olaparib, Rucaparib, or Niraparib), in case the subject has mutations and/or variants in DNA repair genes or ERG gene translocations or TP53 mutations or loss;
> kinase inhibitors, preferably Gefitinib, Capivasertib, or Sorafenib) in case the subject has mutations in protein kinases;
> mTOR inhibitors, preferably Everolimus in case the subject has PTEN loss or PIK3CA mutations; or
> Immune Checkpoint inhibitors, preferably Pembroluzimab or Nivolumab in case the subject has high tumor mutational burden (TMB);

wherein said treatment is optionally further comprises ADT and/or radiotherapy, preferably EBRT;

preferably wherein the ADT is selected from; Luteinizing hormone-releasing hormone (LHRH) agonist alone, LHRH agonists combined with a first or second generation antiandrogen, or LHRH antagonists, preferably wherein the LHRH agonist is selected from Goserelin, Leuprolide, or Triptorelin, and/or the first generation antiandrogen is selected from Nilutamide, Flutamide, or Bicalutamide, and/or the second generation antiandrogen is selected from Apalutamide, Darolutamide, Enzalutamide, and Proxalutamide, and/or the LHRH antagonist is selected from Degarelix or Relugolix.

13. A computer program product comprising instructions which, when the program is executed by a computer, cause the computer to carry out a method for predicting the outcome for a subject suffering from prostate cancer, based on the PDE4D and ABCC5 expression levels determined in a prostate cancer sample obtained from the subject, the method comprising: predicting the outcome for the subject based on the PDE4D and ABCC5 expression levels.

14. Use of a kit to determine an outcome for a subject suffering from prostate cancer, the use comprising

using the kit to determine the expression levels of PDE4D and ABCC5 in a prostate cancer sample obtained from the subject, and

determining the outcome for the subject based on the determined expression levels,

wherein the kit comprises means for determining the expression levels of PDE4D and ABCC5 in a biological sample.

15. Therapy for use in the treatment of prostate cancer in a subject, the use comprising receiving the result of the determined expression levels of PDE4D and ABCC5 in a prostate cancer sample obtained from the subject, predicting the outcome for the subject based on the PDE4D and ABCC5 expression levels, wherein the outcome is a good outcome or a poor outcome, and administering the therapy when a poor outcome is predicted,

wherein the therapy is selected from:

radiation therapy, preferably ERBT, supplemented with ADT and one or more of Abiraterone, Apalutamide or Enzalutamide; or

ADT supplemented with taxane based chemotherapy, preferably docetaxel, and supplemented with one of Abiraterone, Apalutamide or Enzalutamide; or

one or more treatments selected from:

PARP inhibitors, preferably Olaparib, Rucaparib, or Niraparib, in case the subject has mutations and/or variants in DNA repair genes or ERG gene translocations or TP53 mutations or loss;

kinase inhibitors, preferably Gefitinib, Capivasertib, or Sorafenib) in case the subject has mutations in protein kinases;

mTOR inhibitors, preferably Everolimus in case the subject has PTEN loss or PIK3CA mutations; or

Immune Checkpoint inhibitors, preferably Pembroluzimab or Nivolumab in case the subject has high tumor mutational burden (TMB);

wherein said treatment is optionally further comprises ADT and/or radiotherapy, preferably EBRT;
preferably wherein the ADT is selected from; Luteinizing hormone-releasing hormone (LHRH) agonist alone, LHRH agonists combined with a first or second generation antiandrogen, or LHRH antagonists, preferably wherein the LHRH agonist is selected from Goserelin, Leuprolide, or Triptorelin, and/or the first generation antiandrogen is selected from Nilutamide, Flutamide, or Bicalutamide, and/or the second generation antiandrogen is selected from Apalutamide, Darolutamide, Enzalutamide, and Proxalutamide, and/or the LHRH antagonist is selected from Degarelix or Relugolix.

**Fig. 1**

**Fig. 2**

Fig. 3

Fig. 4

Fig. 5

Fig. 6

Fig. 7

Fig. 8

**Fig. 9**

**Fig. 10**

**Fig. 11**

**Fig. 12**

**Fig. 13**

**Fig. 14**

**Fig. 15**

**Fig. 16**

**Fig. 17**

**Fig. 18**

**Fig. 19**

Number at risk
Group: pT2
276  260  252  245  209  168  136   98   45   20    4
Group: pT3a
235  230  225  216  170  132  106   72   39   13    2
Group: pT3b
146  139  127  117   91   68   50   34   17    8    3
Group: pT3c, and pT4
117  117  117  114   96   66   55   37   20    9    5

**Fig. 20**

**Fig. 21**

**Fig. 22**

Fig. 23

Fig. 24

**Fig. 25**

**Fig. 26**

Fig. 27

Fig. 28

PCa_death_class

## Fig. 29

## Fig. 30

Fig. 31

Fig. 32

Number at risk
Group: PDE4D_ABCC5_high_EAU_high
133 132 128 122 93 67 51 33 17 7 5
Group: PDE4D_ABCC5_high_EAU_low
230 230 228 224 180 128 99 68 37 16 6
Group: PDE4D_ABCC5_low_EAU_high
127 125 116 104 75 57 40 24 10 3 1
Group: PDE4D_ABCC5_low_EAU_low
98 98 97 97 83 55 36 27 15 8 6

# Fig. 33

Number at risk
Group: PDE4D_ABCC5_high_EAU_high
120 119 115 110 85 59 44 27 13 6 4
Group: PDE4D_ABCC5_high_EAU_low
172 172 170 166 133 93 72 48 29 12 3
Group: PDE4D_ABCC5_low_EAU_high
123 121 112 100 72 54 37 21 9 2 0
Group: PDE4D_ABCC5_low_EAU_low
88 88 87 87 74 48 33 24 13 6 5

# Fig. 34

Fig. 35

Fig. 36

Fig. 37

Fig. 38

**Fig. 39**

Number at risk
Group: PDE4D_ABCC5_high_EAU_high
112 108 97 81 51 39 18 9 5 2 2
Group: PDE4D_ABCC5_high_EAU_low
151 130 107 84 57 38 20 12 7 3 2
Group: PDE4D_ABCC5_low_EAU_high
115 100 88 68 47 27 19 11 5 0 0
Group: PDE4D_ABCC5_low_EAU_low
79 69 60 51 36 24 13 12 8 6 3

**Fig. 40**

Number at risk
Group: PDE4D_ABCC5_high_EAU_high
42 35 28 23 14 9 5 2 1 0
Group: PDE4D_ABCC5_high_Risk_low
41 33 23 17 13 9 5 1 1 0
Group: PDE4D_ABCC5_low_Risk_high
74 65 51 43 31 23 13 6 2 0
Group: PDE4D_ABCC5_low_Risk_low
27 22 15 12 9 3 2 2 0 0

**Fig. 41**

**Fig. 42**

**EUROPEAN SEARCH REPORT**

Application Number

EP 24 21 8311

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 960 876 A1 (KONINKLIJKE PHILIPS NV [NL]) 2 March 2022 (2022-03-02) * para. 12, 13, 22, 44, 57, 61, 63, 64, 70, 72-75, 89, 101, 102, 105, 109, 123; claims 1, 4, 5, 9, 11; fig. 4 * | 1-15 | INV. C12Q1/6886 |
| X | EP 4 357 462 A1 (KONINKLIJKE PHILIPS NV [NL]) 24 April 2024 (2024-04-24) * para. 7, 60, 63, 184-190, 200, 207-210, 223; claim 1, 6; fig. 4 * | 1-15 | |
| A | SRIGLEY JOHN R. ET AL: "One is the new six: The International Society of Urological Pathology (ISUP) patient-focused approach to Gleason grading", CANADIAN UROLOGICAL ASSOCIATION JOURNAL = JOURNAL DE L'ASSOCIATION DES UROLOGUES DU CANADACANADAOCT 2012, vol. 10, no. 9-10, 13 October 2016 (2016-10-13), page 339, XP093277435, Canadian Urological Association journal = Journal de l'Association des urologues du CanadaCanadaOct 2012 ISSN: 1920-1214, DOI: 10.5489/cuaj.4146 Retrieved from the Internet: URL:https://pmc.ncbi.nlm.nih.gov/articles/PMC5085914/pdf/cuaj-9-10-339.pdf> * the whole document * -/-- | 1-15 | TECHNICAL FIELDS SEARCHED (IPC) C12Q |

The present search report has been drawn up for all claims

| Place of search | Date of completion of the search | Examiner |
|---|---|---|
| Munich | 19 May 2025 | Ripaud, Leslie |

## EUROPEAN SEARCH REPORT

Europäisches
Patentamt
European
Patent Office
Office européen
des brevets

**Application Number**

EP 24 21 8311

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Teo Min Yuen ET AL: "Treatment of Advanced Prostate Cancer", Annual review of medicine, 1 January 2019 (2019-01-01), pages 479-499, XP093277514, DOI: 10.1146/annurev-med-051517- Retrieved from the Internet: URL:https://www.annualreviews.org/docserver/fulltext/med/70/1/annurev-med-051517-011947.pdf?expires=1747231592&id=id&accname=ar-367370&checksum=E0418F8411B74D6F1145BB492DEC8D76 * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 19 May 2025 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 8311

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2025

| Patent document cited in search report | | Publication date | Patent family member(s) | | Publication date |
|---|---|---|---|---|---|
| EP 3960876 | A1 | 02-03-2022 | CN | 116802312 A | 22-09-2023 |
| | | | EP | 3960876 A1 | 02-03-2022 |
| | | | EP | 4204579 A1 | 05-07-2023 |
| | | | JP | 2023538868 A | 12-09-2023 |
| | | | US | 2023323469 A1 | 12-10-2023 |
| | | | WO | 2022043120 A1 | 03-03-2022 |
| EP 4357462 | A1 | 24-04-2024 | EP | 4357462 A1 | 24-04-2024 |
| | | | WO | 2024083612 A1 | 25-04-2024 |

EPO FORM P0459

For more details about this annex : see Official Journal of the European Patent Office, No. 12/82

**REFERENCES CITED IN THE DESCRIPTION**

**Patent documents cited in the description**

- WO 2019122037 A1

**Non-patent literature cited in the description**

- Cancer Incidence and Mortality Worldwide: IARC CancerBase. **FERLAY J et al.** GLOBOCAN. International Agency for Research on Cancer, 2012
- **RODRIGUES G. et al.** Pre-treatment risk stratification of prostate cancer patients: A critical review. *Canadian Urological Association Journal*, 2012, vol. 6 (2), 121-127
- **MOHLER J. et al.** NCCN clinical practice guidelines in oncology: Prostate cancer, Version 1.2016. *Journal of the National Comprehensive Cancer Network*, 2016, vol. 14 (1), 19-30
- **HERNANDEZ D.J. et al.** Contemporary evaluation of the D'Amico risk classification of prostate cancer. *Journal of Urology*, 2007, vol. 70 (5), 931-935
- **JUNG J.W. et al.** Stratification of patients with intermediate-risk prostate cancer. *BJU International*, 2015, vol. 115 (6), 907-912
- **ABERN M.R. et al.** Delayed radical prostatectomy for intermediate-risk prostate cancer is associated with biochemical recurrence: Possible implications for active surveillance from the SEARCH database. *The Prostate*, 2013, vol. 73 (4), 409-417
- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, 1987
- Methods in Enzymology. Academic Press
- Computational Molecular Biology. Oxford University Press, 1988
- Biocomputing: Informatics And Genome Projects. Academic Press, 1993
- Computer Analysis Of Sequence Data, Part I. Humana Press, 1994
- **VON HEINJE, G.** Sequence Analysis In Molecular Biology. Academic Press, 1987
- Sequence Analysis Primer. M Stockton Press, 1991
- **CARILLO, H.** ; **LIPTON, D.** *SIAM J. Applied Math*, 1988, vol. 48, 1073
- Guide To Huge Computers. Academic Press, 1994
- **CARILLO, H.** ; **LIPTON, D.** *Siam J. Applied Math*, 1988, vol. 48, 1073
- **DEVEREUX, J. et al.** *Nucleic Acids Research*, 1984, vol. 12 (1), 387
- **ATSCHUL, S. F. et al.** *J. Molec. Biol.*, 1990, vol. 215, 403
- **SRIGLEY et al.** *Urol Assoc J.*, September 2016, vol. 10 (9-10), 339-341